# FASCICULE DE BREVET EUROPEEN

(11) **EP 1 774 990 B1**
(45) Date de publication et mention de la délivrance du brevet: **09.12.2009**
(21) Numéro de dépôt: 06291560.8
(22) Date de dépôt: 06.10.2006
(51) Int. Cl.: A61Q 19/02, A61K 8/60

(54) **Utilisation de composés C-glycosides pour dépigmenter la peau**
Verwendung von C-Glycosiden zur Depigmentierung der Haut
Use of c-glycosides for depigmenting skin

(30) Priorité: 11.10.2005 FR 0553080; 03.07.2006 FR 0652774
(43) Date de publication de la demande: 18.04.2007
(73) Titulaire: L'Oréal, 75008 Paris (FR)
(72) Inventeur: Rolland, Anne, 75017 Paris (FR)
(74) Mandataire: Kromer, Christophe

(56) Documents cités:
- EP-A1- 0 754 449
- WO-A-02/051828
- US-A- 4 454 123
- US-A- 5 310 730
- US-A- 5 786 469

## Description

La présente invention se rapporte à l'utilisation de composé C-glycoside pour dépigmenter et/ou blanchir la peau.

La couleur de la peau humaine est fonction de différents facteurs et notamment des saisons de l'année, de la race et du sexe, elle est principalement déterminée par la nature et la concentration de mélanine produite par les mélanocytes. Les mélanocytes sont les cellules spécialisées qui, par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc), à défaut de pouvoir repigmenter la peau lésée, on achève de dépigmenter les zones de peau normale résiduelle pour donner à l'ensemble de la peau une teinte blanche homogène.

Le mécanisme de formation de la pigmentation de la peau, c'est-à-dire de la formation de la mélanine est particulièrement complexe et fait intervenir schématiquement les principales étapes suivantes :
**Tyrosine --- > Dopa --> Dopaquinone ---> Dopachrome ---> Mélanine**

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Les substances dépigmentantes peuvent agissent directement sur la vitalité des mélanocytes épidermiques où se déroule la mélanogénèse et/ou interférer avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés peuvent cependant engendrer des effets indésirables comme l'apparition de rougeurs cutanées dans des situations particulières telles qu'une utilisation à des concentrations importantes, une peau sensible ou présentant un désordre dermatologique...

Par ailleurs, on utilise couramment, comme inhibiteur de l'activation de la tyrosinase, l'acide kojique qui complexe le cuivre présent dans le site actif de cette enzyme. Cependant, ce composé peut s'avérer instable en solution, ce qui complique sa formulation.

Il subsiste le besoin de nouveaux agents blanchissants de la peau humaine à action aussi efficace que ceux connus et non irritants, non toxiques et/ou non allergisants pour la peau, tout en étant stable dans une composition, ou bien alternativement qui possède une action renforcée de façon à pouvoir être utilisé en quantité plus faible, ce qui diminue considérablement les effets secondaires observés.

A cet égard la Demanderesse a de manière surprenante et inattendue découvert que certains composés C-glycosides présentaient une bonne activité dépigmentante sans présenter de cytotoxicité.

Les C-glycosides ont déjà été décrits dans l'EP 1 345 919 comme ayant la propriété d'induire la synthèse de protéoglycannes et de glycosaminoglycannes contribuant ainsi à renforcer la matrice extra-cellulaire du derme.

De façon plus précise, l'invention a donc pour objet l'utilisation cosmétique ou pharmaceutique, en particulier dermatologique, d'au moins un composé de formule (I) suivante : 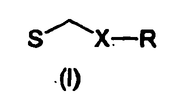 dans laquelle,
- R désigne un radical linéaire en C₁-C₄, éventuellement substitué par -OH, -COOH ou - COOR"₂, R"₂ étant un radical alkyle saturé en C₁-C₄,
- X représente un radical choisi parmi les groupements : 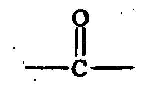 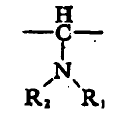 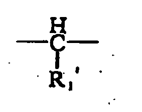 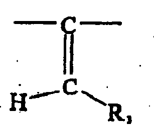 avec R₁, R₂ et R₃ représentant, indépendamment l'un de l'autre, un atome d'hydrogène, ou un radical R, avec R tel que défini précédemment, et R'₁ représente un atome d'hydrogène, un groupe -OH ou un radical R, avec R tel que défini précédemment, R1 pouvant désigner également un radical aryle en C₆ à C₁₀
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être α ou β, ainsi que leurs sels cosmétiquement acceptables, leurs solvates tels que les hydrates, en tant qu'agent blanchissant de la peau et/ou comme agent anti-brunissement.

Dans le cadre de la présente invention, par « halogène », on entend le chlore, le fluor, le brome ou l'iode.

Par « hétéroatomes », on entend l'azote, l'oxygène ou le soufre.

Le terme « aryle » désigne un cycle aromatiques, à l'exception du phényle, éventuellement substitué par un ou plusieurs radicaux alkyles en C1-C4.

Le terme « cycloalkyle en C₃ à C₈ » désigne un cycle aliphatique ayant de 3 à 8 atomes de carbone, incluant par exemple le cyclopropyle, le cyclopentyle et le cyclohéxyle.

Parmi les groupes alkyle convenant à la mise en oeuvre de l'invention, on peut notamment citer les groupes méthyle, éthyle, isopropyle, n-propyle, n-butyle, t-butyle, isobutyle, sec-butyle, pentyle, n-hexyle, cyclopropyle, cyclopentyle, cyclohexyle, et allyle.

Selon un mode de réalisation de l'invention, on peut utiliser un dérivé C-glycoside répondant à la formule (I) pour lequel S peut représenter un monosaccharide ou un polysaccharide contenant jusqu'à 6 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine obligatoirement protégée, X et R conservant par ailleurs l'ensemble des définitions précédemment données.

Avantageusement, un monosaccharide de l'invention peut être choisi parmi le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et en particulier le D-xylose.

Plus particulièrement, un polysaccharide de l'invention contenant jusqu'à 6 unités sucre peut être choisi parmi le D-maltose, le D-lactose, le D-cellobiose, le D-maltotriose, un disaccharide associant un acide uronique choisi parmi l'acide D-iduronique ou l'acide D-glucuronique avec une hexosamine choisie parmi la D-galactosamine, la D-glucosamine, la N-acétyl-D-galactosamine, la N-acétyl-D-glucosamine, un oligosaccharide contenant au moins un xylose qui peut être avantageusement choisi parmi le xylobiose, le méthyl-β-xylobioside, le xylotriose, le xylotétraose, le xylopentaose et le xylohexaose et notamment le xylobiose qui est composé de deux molécules de xylose liées par une liaison 1-4.

Plus particulièrement, S peut représenter un monosaccharide choisi parmi le D-glucose, le D-xylose, le D-fucose, le D-galactose, le D-maltose et notamment le D-xylose.

Selon un autre mode de réalisation de l'invention, on peut utiliser des dérivés C-glycoside répondant à la formule (I) pour lesquels X représente un groupement choisi parmi -CO-, - CH(OH)-, -CH(NR₁R₂)-, -CH(R)-, en particulier -CO-, -CH(OH)-, -CH(NH₂)-, - CH(NHCH₂CH₂CH₂OH)-, -CH(NHPh)-, -CH(CH₃)-, et plus particulièrement un groupement - CO-, -CH(OH)-, -CH(NH₂)-, et préférentiellement un groupement -CH(OH)-, S et R conservant par ailleurs l'ensemble des définitions précédemment données.

Selon un autre mode de réalisation de l'invention, on peut utiliser un dérivé C-glycoside répondant à la formule (I) pour lesquels R désigne un radical linéaire en C₁-C₃, éventuellement substitué par -OH, -COOH ou -COOR"₂, R"₂ étant un radical alkyle saturé en C,-C₄, notamment éthyl. Préférentiellement R désigne un radical alkyle linéaire non substitué en C₁-C₄, notamment C₁-C₂, en particulier éthyle.

De préférence, on utilise un dérivé C-glycoside de formule (I) pour lesquels :
- R désigne un radical linéaire en C₁-C₄, notamment C₁-C₃, éventuellement substitué par - OH, -COOH ou -COOR"₂, R"₂ étant un radical alkyle saturé en C₁-C₄, notamment éthyle ;
- S représente un monosaccharide comme décrit précédemment ;
- X représente un groupement choisi parmi -CO-, -CH(OH)-, -CH(NH₂)-,
- CH(NHCH₂CH₂CH₂OH)-, -CH(NHPh)-. -CH(CH₃)-, et plus particulièrement un groupement - CO-. -CH(OH)-, -CH(NH₂)-, et préférentiellement un groupement-CH(OH)-.

Préférentiellement, on utilise un dérivé C-glycoside de formule (I) pour lesquels :
- R désigne un radical alkyle linéaire non substitué en C₁-C₄, notamment C₁-C₂, en particulier éthyle ;
- S représente un monosaccharide comme décrit précédemment ; notamment le D-glucose, le D-xylose, la N-acétyl-D-glucosamine ou le L-fucose, et en particulier le D-xylose ;
- X représente un groupement choisi parmi -CO-, -CH(OH)-, -CH(NH₂)-, et préférentiellement un groupement -CH(OH)-.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Parmi les dérivés C-glycosides de formule (I), utilisés selon l'invention, on préfère tout particulièrement :
1. C-β-D-xylopyranoside-n-propane-2-one ;
2. C-α-D-xylopyranoside-n-propane-2-one ;
3. 1-[2-(3-hydroxy-propylamino)-propyl]-C-β-D-xylopyranose ;
4. 1-[2-(3-hydroxy-propylamino)-propyl]-C-α-D-xylopyranose ;
5. C-β-D-xytopyranoside-2-hydroxy-propane ;
6. C-α-D-xylopyranoside-2-hydroxy-propane ;
7. C-β-D-xytopyranoside-2-amino-propane ;
8. C-α-D-xylopyranoside-2-amino-propane ;
9. C-β-D-xylopyranoside-2-phénylamino-propane ;
10. C-α-D-xylopyranoside-2-phénylamino-propane ;
11. ester éthylique de l'acide 3-méthyl-4-(C-β-D-xylopyranoside)-butyrique ;
12. ester éthylique de l'acide 3-méthyl-4-(C-α-D-xylopyranoside)-butyrique ;
13. acide 6-(C-β-D-xylopyranoside)-5-céto-hexanoique ;
14. acide 6-(C-α-D-xylopyranoside)-5-céto-hexanoique ;
15. acide 6-(C-β-D-xylopyranoside)-5-hydroxy-hexanoique ;
16. acide 6-(C-α-D-xylopyranoside)-5-hydroxy-hexanoique ;
17. acide 6-(C-β-D-xylopyranoside)-5-amino-hexanoique ;
18. acide 6-(C-α-D-xylopyranoside)-5-amino-hexanoique ;
19. acide 6-(C-β-D-xylopyranoside)-5-phenylamino-hexanoique ;
20. acide 6-(C-α-D-xylopyranoside)-5-phenylamino-hexanoique ;
21. 1-(C-β-D-xylopyranoside)-hexane-2,6-diol ;
22. 1-(C-α-D-xylopyranoside)-hexane-2,6-diol ;
23. acide 5-(C-β-D-xylopyranoside)-4-céto-pentanoique ;
24. acide 5-(C-α-D-xylopyranoside)-4-céto-pentanoique ;
25. acide 5-(C-β-D-xylopyranoside)-4-hydroxy-pentanoique ;
26. acide 5-(C-α-D-xylopyranoside)-4-hydroxy-pentanoique ;
27. acide 5-(C-β-D-xylopyranoside)-4-amino-pentanoique ;
28. acide 5-(C-α-D-xylopyranoside)-4-amino-pentanoique ;
29. acide 5-(C-β-D-xylopyranoside)-4-phénylamino-pentanoique ;
30. acide 5-(C-α-D-xylopyranoside)-4-phénylamino-pentanoique ;
31. 1-(C-β-D-xylopyranoside)-pentane-2,5-diol ;
32. 1-(C-α-D-xylopyranoside)-pentane-2,5-diol ;
33. 1-(C-β-D-fucopyranoside)-propane-2-one ;
34. 1-(C-α-D-fucopyranoside)-propane-2-one ;
35. 1-(C-β-L-fucopyranoside)-propane-2-one ;
36. 1-(C-α-L-fucopyranoside)-propane-2-one ;
37. 1-(C-β-D-fucopyranoside)-2-hydroxy-propane ;
38. 1-(C-α-D-fucopyranoside)-2-hydroxy-propane ;
39. 1-(C-β-L-fucopyranoside)-2-hydroxy-propane;
40. 1-(C-α-L-fucopyranoside)-2-hydroxy-propane ;
41. 1-(C-β-D-fucopyranoside)-2-amino-propane;
42. 1-(C-α-D-fucopyranoside)-2-amino-propane ;
43. 1-(C-β-L-fucopyranoside)-2-amino-propane ;
44. 1-(C-α-L-fucopyranoside)-2-amino-propane ;
45. 1-(C-β-D-fucopyranoside)-2-phénylamino-propane ;
46. 1-(C-α-D-fucopyranoside)-2-phénylamino-propane ;
47. 1-(C-β-L-fucopyranoside)-2-phénylamino-propane ;
48. 1-(C-α-L-fucopyranoside)-2-phénylamino-propane ;
49. ester éthylique de l'acide 3-méthyl-4-(C-β-D-fucopyranoside)-butyrique ;
50. ester éthylique de l'acide 3-méthyl-4-(C-α-D-fucopyranoside)-butyrique ;
51. ester éthylique de l'acide 3-méthyl-4-(C-β-L-fucopyranoside)-butyrique ;
52. ester éthylique de l'acide 3-méthyl-4-(C-α-L-fucopyranoside)-butyrique ;
53. acide 6-(C-β-D-fucopyranoside)-5-céto-hexanoique ;
54. acide 6-(C-α-D-fucopyranoside)-5-céto-hexanoique ;
55. acide 6-(C-β-L-fucopyranoside)-5-céto-hexanoique ;
56. acide 6-(C-α-L-fucopyranoside)-5-céto-hexanoique ;
57. acide 6-(C-β-D-fucopyranoside)-5-hydroxy-hexanoique ;
58. acide 6-(C-α-D-fucopyranoside)-5-hydroxy-hexanoique ;
59. acide 6-(C-β-L-fucopyranoside)-5-hydroxy-hexanoique ;
60. acide 6-(C-α-L-fucopyranoside)-5-hydroxy-hexanoique ;
61. acide 6-(C-β-D-fucopyranoside)-5-amino-hexanoique ;
62. acide 6-(C-α-D-fucopyranoside)-5-amino-hexanoique ;
63. acide 6-(C-β-L-fucopyranoside)-5-amino-hexanoique ;
64. acide 6-(C-α-L-fucopyranoside)-5-amino-hexanoique ;
65. 1-(C-β-D-fucopyranoside)-hexane-2,6-diol ;
66. 1-C-α-D-fucopyranoside)-hexane-2,6-diol ;
67. 1-(C-β-L-fucopyranoside)-hexane-2,6-diol ;
68. 1-(C-α-L-fucopyranoside)-hexane-2,6-diol ;
69. acide 5-(C-β-D-fucopyranoside)-4-céto-pentanoique ;
70. acide 5-(C-α-D-fucopyranoside)-4-céto-pentanoique ;
71. acide 5-(C-β-L-fucopyranoside)-hexane-2,6-diol)-4-céto-pentanoique ;
72. acide 5-(C-α-L-fucopyranoside)-hexane-2,6-diol)-4-céto-pentanoique ;
73. acide 5-(C-β-D-fucopyranoside)-4-hydroxy-pentanoique ;
74. acide 5-(C-α-D-fucopyranoside)-4-hydroxy-pentanoique ;
75. acide 5-(C-β-L-fucopyranoside)-4-hydroxy-pentanoique ;
76. acide 5-(C-α-L-fucopyranoside)-4-hydroxy-pentanoique ;
77. acide 5-(C-β-D-fucopyranoside)-4-amino-pentanoique ;
78. acide 5-(C-α-D-fucopyranoside)-4-amino-pentanoique
79. acide 5-(C-β-L-fucopyranoside)-4-amino-pentanoique
80. acide 5-(C-α-L-fucopyranoside)-4-amino-pentanoique ;
81. 1-(C-β-D-fucopyranoside)-pentane-2,5-diol ;
82. 1-(C-α-D-fucopyranoside)-pentane-2,5-diol ;
83. 1-(C-β-L-fucopyranoside)-pentane-2,5-diol ;
84. 1-(C-α-L-fucopyranoside)-pentane-2,5-diol ;
85. 1-(C-β-D-Glucopyranosyl)-2-hydroxyl-propane ;
86. 1-(C-α-D-Glucopyranosyl)-2-hydroxyl-propane ;
87. 1-(C-β-D-Glucopyranosyl)-2-amino-propane;
88. 1-(C-α-D-Glucopyranosyl)-2-amino-propane ;
89. 1-(C-β-D-Glucopyranosyl)-2-phénylamino-propane ;
90. 1-(C-α-D-Glucopyranosyl-2-phénylamino-propane ;
91. ester éthylique de l'acide 3-méthyl-4-(C-β-D-Glucopyranosyl)-butyrique ;
92. ester éthylique de l'acide 3-méthyl-4-(C-β-D-Glucopyranosyl)-butyrique ;
93. acide 6-(C-β-D-Glucopyranosyl)-5-céto-hexanoique ;
94. acide 6-(C-α-D-Glucopyranosyl)-5-céto-hexanoique ;
95. acide 6(C-β-D-Glucopyranosyl)-5-hydroxy-hexanoique ;
96. acide 6-(C-α-D-Glucopyranosyl)-5-hydroxy-hexanoique :
97. acide 6-(C-β-D-Glucopyranosyl)-5-amino-hexanoique ;
98. acide 6-(Cα-D-Glucopyranosyl)-5-amino-hexanoique ;
99. acide 6-(C-β-D-Glucopyranosyl)-5-phénylamino-hexanoique ;
100. acide 6-(C-α-D-Glucopyranosyl)-5-phénylamino-hexanoique ;
101. 1-(C-β-D-Glucopyranosyl)hexane-2,6-diol ;
102. 1-(C-α-D-Glucopyranosyl)-hexane-2,6-diol ;
103. acide 6(C-β-D-Glucopyranosyl)-5-céto-pentanoique ;
104. acide 6-(C-α-D-Glucopyranosyl)-5-céto-pentanoique,
105. acide 6-(C-β-D-Glucopyranosyl)-5-hydroxy-pentanoique ;
106. acide 6-(C-α-D-Glucopyranosyl)-5-hydroxy-pentanoique ;
107. acide 6(C-β-D-Glucopyranosyl)-5-amino-pentanoique ;
108. acide 6-(C-α-D-Glucopyranosyl)-5-hydroxy-pentanoique ;
109. acide 6-(C-β-D-Glucopyranosyl)-5-phénylamino-pentanoique ;
110. acide 6-(C-α-D-Glucopyranosyl)-5-phénylamino-pentanoique ;
111. 1-(C-β-D-Glucopyranosyl)-pentane-2,5-diol ;
112. 1-(C-α-D-Glucopyranosyl)-pentane-2,5-diol ;
113. 1-(C-β-D-galactopyranosyl)-2-hydroxyl-propane ;
114. 1-(C-α-D-galactopyranosyl)-2-hydroxyl-propane ;
115. 1-(C-β-D-galactopyranosyl)-2-amino-propane ;
116. 1-(C-α-D-galactopyranosyl)-2-amino-propane ;
117. 1-(C-β-D-galactopyranosyl)-2-phénylamino-propane;
118. 1-(C-α-D-galactopyranosyl)-2-phénylamino-propane ;
119. ester éthylique de l'acide 3-méthyl-4-(C-β-D-galadopyranosyl)-butyrique ;
120. ester éthylique de l'acide 3-méthyl-4-(C-α-D-galactopyranosyl)-butyrique ;
121, acide 6-(C-β-D-galactopyranosyl)-5-céto-hexanoique ;
122. acide 6-(C-α-D-galactopyranosyl)-5-céto-hexanoique ;
123. acide 6(C-β-D-galactopyranosyl)-5-hydroxy-hexanoique ;
124. acide 6(C-α-D-galactopyranosyl)-5-hydroxy-hexanoique ;
125. acide 6(C-β-D-galactopyranosyl)-5-amino-hexanoique ;
126. acide 6-(C-α-D-galactopyranosyl)-5-amino-hexanoique
127. acide 6-(C-β-D-galactopyranosyl)5-phénylamino-hexanoique ;
128. acide 6-(C-α-D-galactopyranosyl)5-phénylamino-hexanoique ;
129. 1-(C-β-D-galactopyranosyl)-hexane-2,6-diol ;
130. 1-(C-α-D-galactopyranosyl)-hexane-2,6-diol ;
131. acide 6-(C-β-D-galactopyranosyl)-5-céto-pentanoique ;
132. acide 6-(C-α-D-galactopyranosyl)-5-céto-pentanoique ;
133. acide 6-(C-β-D-galactopyranosyl)-5-hydroxy-pentanoique ;
134. acide 6-(C-α-D-galactopyranosyl)-5-hydroxy-pentanoique ;
135. acide 6-(C-β-D-galactopyranosyl)-5-amino-pentanoique ;
136. acide 6-(C-α-D-galactopyranosyl)-5-amino-pentanoique ;
137. acide 6-(C-β-D-galactopyranosyl)-5-phénylamino-pentanoique ;
138. acide 6-(C-α-D-galactopyranosyl)-5-phénylamino-pentanoique ;
129. 1-(C-β-D-galactopyranosyl)-pentane-2,6-diol ;
140. 1-(C-α-D-galactopyranosyl)-pentane-2,6-diol ;
141. 1-(C-β-D-fucofuranosyl)-propane-2-one ;
142. 1-(C-α-D-fucofuranosyl)-propane-2-one ;
143. 1-(C-β-L-fucofuranosyl)-propane-2-one ;
144. 1-(C-α-L-fucofuranosyl)-propane-2-one ;
145. 3'-(acétamido-C-β-D-glucopyranosyl)-propane-2'-one ;
146. 3'-(acétamido-C-α-D-glucopyranosyl)-propane-2'-one ;
147. 1-(C-β-D-galactopyranosyl)-2-hydroxyl-propane ;
148. 1-(C-α-D-galactopyranosyl)-2-amino-propane ;
149. 1-(acétamido-C-β-D-glucopyranosyl)-2-phénylamino-propane ;
150. 1-(acétamido-C-α-D-glucopyranosyl)-2-phénylamino-propane ;
151. ester éthylique de l'acide 3-méthyl-4-(acétamido-C-β-D-glucopyranosyl)-butyrique ;
152. ester éthylique de l'acide 3-méthyl-4-(acétamido-C-α-D-glucopyranosyl)-butyrique ;
153. acide 6-(acétamido-C-β-D-glucopyranosyl)-5-céto-hexanoique ;
154. acide 6-(acétamido-C-α-D-glucopyranosyl)-5-céto-hexanoique ;
155. acide 6-(acétamido-C-β-D-glucopyranosyl)-5-hydroxy-hexanoique ;
156. acide 6-(acétamido-C-α-D-glucopyranosyl)-5-hydroxy-hexanoique ;
157. acide 6-(acétamido-C-β-D-glucopyranosyl)-5-amino-hexanoique ;
158. acide 6-(acétamido-C-α-D-glucopyranosyl)-5-amino-hexanoique ;
159. acide 6-(acétamido-C-β-D-glucopyranosyl)5-phénylamino-hexanoique ;
160. acide 6-(acétamido-C-α-D-glucopyranosyl)5-phénylamino-hexanoique ;
161. 1-(acétamido-C-β-D-glucopyranosyl)-hexane-2,6-diol ;
162. 1-(acétamido-C-α-D-glucopyranosyl)-hexane-2,6-diol ;
163. acide 6-(acétamido-C-β-D-glucopyranosyl)-5-céto-pentanoique ;
164. acide 6-(acétamido-C-α-D-glucopyranosyl)-5-céto-pentanoique ;
165. acide 6-(acétamido-C-β-D-glucopyranosyl)-5-hydroxy-pentanoique ;
166. acide 6-(acétamido-C-α-D-glucopyranosyl)-5-hydroxy- pentanoique ;
167. acide 6-(acétamido-C-β-D-glucopyranosyl)-5-amino- pentanoique ;
168. acide 6-(acétamido-C-α-D-glucopyranosyl)-5-amino- pentanoique ;
169. acide 6-(acétamido-C-β-D-glucopyranosyl)-5-phénylamino- pentanoique ;
170. acide 6-(acétamido-C-α-D-glucopyranosyl)-5-phénylamino- pentanoique ;
171. 1-(acétamido-C-β-D-glucopyranosyl)-pentane-2,5-diol ;
172. 1-(acétamido-C-α-D-glucopyranosyl)-pentane-2,5-diol.

On utilisera de préférence le C-α-D-xylopyranoside-2-hydroxy-propane et le C-β-D-xylopyranoside-2-hydroxy-propane, de préférence le C-β-D-xylopyranoside-2-hydroxy-propane.

Bien entendu, selon l'invention, les dérivés C-glycosides répondant à la formule (I) peuvent être utilisés seuls ou en mélange et en toute proportion.

Selon l'invention, les dérivés C-glycosides répondant à la formule (I) peuvent être d'origine naturelle ou synthétique, totalement ou partiellement purifiés ou toute préparation les contenant.

Par origine naturelle, on entend un dérivé extrait de matériel naturel dans lequel il se trouve présent, par exemple des plantes. Par origine synthétique, on entend un dérivé préparé par synthèse chimique ou par biotechnologie.

L'expression " totalement ou partiellement purifiés " signifie ici que, durant sa synthèse ou par rapport à son état naturel (plante ou cellules fraîches ou desséchées), le dérivé C-glycoside répondant à la formule (I) dans la composition de l'invention, a été concentré et/ou a été débarrassé, respectivement d'au moins une partie des produits réactionnels secondaires issus de sa synthèse ou d'au moins une partie des autres constituants du matériel naturel dans lequel il se trouve présent.

Les dérivés C-glycosides peuvent notamment être obtenus par la méthode de synthèse décrite dans le document EP 1 345 919.

Les composés de formule (I) selon l'invention permettent de dépigmenter et/ou d'éclaircir efficacement la peau d'êtres humains.

La pigmentation de la peau est un processus physiologique normal résultant de l'exposition de la peau aux rayons du soleil.
Il peut, dans un but esthétique, être souhaitable d'embellir l'aspect de la peau en limitant la pigmentation et ainsi diminuer l'apparition de zones de peau plus foncées.

Il arrive également que la pigmentation résulte de désordres cutanés pouvant, par exemple, être liés à une prolifération locale de mélanocytes actifs.

Les C-glycosides sont notamment destinés à être appliqués sur la peau d'individus présentant des taches de pigmentation brunâtres, des taches de sénescence, ou sur la peau d'individus désirant combattre l'apparition d'une couleur brunâtre provenant de la mélanogénèse, par exemple à la suite d'une exposition aux rayonnements ultra-violet.

Ainsi, les composés de formule (I) peuvent être utilisés comme agent blanchissant de la peau et/ou comme agent anti-brunissement, notamment pour prévenir la formation et/ou atténuer les taches pigmentaires, les éphélides, les taches de sénescence et/ou pour éclaircir et/ou blanchir et/ou uniformiser la couleur d'une peau brunie.

Ces composés sont particulièrement efficaces pour prévenir et/ou traiter les taches actiniques. Les taches actiniques, encore appelées lentigo sénile, sont caractérisées par des macules brunes circonscrites correspondant une production locale de mélanine plus importante induite par l'exposition solaire chronique. Elles se rencontrent généralement au niveau du visage, du dos des mains, des avant-bras, du haut du dos et du décolleté, voire le cuir chevelu des zones du scalp dépourvues de cheveux.

L'invention a aussi pour objet l'utilisation d'un composé de formule (I) tel décrit précédemment pour la fabrication d'une composition dermatologique destinée à traiter les désordres pigmentaires de la peau.

Les composés de formule (I) peuvent également être utiles pour traiter les hyperpigmentations régionales par hyperactivité mélanocytaire telles que le mélasma des avant-bras, les mélasmas idiopathiques, survenant lors de la grossesse ("masque de grossesse" ou chloasma) ou d'une contraception oestro-progestative, les puva-lentigines, l'hyperpigmentation post-inflammatoire, les hyperpigmentations accidentelles, éventuellement dues à la photosensibilisation ou à la cicatrisation post-lésionnelle, ainsi que certaines leucodermies, telles que le vitiligo.

Les substances dépigmentantes trouvent également une application dans le blanchiment des phanères, en particulier des poils qu'il peut être souhaitable d'éclaircir afin de les rendre moins visibles.

Que les composés de formule (I) soient utilisés à des fins cosmétique ou pharmaceutique, leur administration peut se faire par différentes voies, par exemple, la voie orale, ces composés seront alors formulés dans des compositions adaptées à ce mode d'administration.
Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules. En particulier, le(s) actif(s) selon l'invention peuvent être incorporés dans toute autre forme de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

L'invention a donc également pour objet un procédé cosmétique de blanchiment de la peau humaine et/ou du cuir chevelu et/ou des muqueuses comprenant l'ingestion ou l'application sur la peau et/ou du cuir chevelu et/ou des muqueuses d'au moins un composé C-glycoside.
Dans le cas d'une application topique, le composé C-glycoside pourra être laissée en contact avec la peau et/ou du cuir chevelu et/ou des muqueuses, puis être éventuellement rincée.
Le procédé convient notamment pour éliminer les taches pigmentaires brunâtres et/ou les taches de sénescence, et/ou pour éclaircir la peau brunie.

En vue de leur utilisation topique, les composés de formule (I) pourront être formulés dans une composition comprenant un milieu physiologiquement acceptable.
En particulier la composition est adaptée à une application topique sur la peau. Le milieu physiologiquement acceptable sera préférentiellement un milieu cosmétiquement ou dermatologiquement acceptable, c'est-à-dire sans aspect désagréable, et qui ne génère pas de picotement, tiraillement ou rougeur inacceptable pour l'utilisateur.

Par milieu physiologiquement acceptable, on comprend un milieu compatible avec les matières kératiniques d'êtres humains comme la peau, les muqueuses, les ongles, le cuir chevelu et/ou les cheveux.

La composition selon l'invention peut être destinée à une application cosmétique ou pharmaceutique, particulièrement dermatologique.

La quantité de composés de formule (I) utilisable dans le cadre de l'invention dépend bien évidemment de l'effet recherché.
A titre d'exemple et pour une administration par voie topique, cette quantité peut aller par exemple de 0,0001% à 25% en poids, 0,001% à 10% en poids, de préférence de 0,01% à 5% en poids, notamment de 0,1 à 2% en poids, par rapport au poids total de la composition.
Dans le cas d'une administration par voie orale, la quantité de composé de formule (I) peut être comprise entre 10 et 1000 mg poids / kg de poids corporel / jour, de préférence 100 mg poids / kg de poids corporel / jour.

Dans le cas d'une administration orale, la composition pourra se présenter sous forme de comprimés, de gélules, de dragées, de sirops, de suspensions, de solutions, de poudres, de granulés, d'émulsions, de suspensions de microsphères ou nanosphères ou vésicules lipidiques ou polymériques permettant une libération contrôlée. De préférence, la composition se présente sous forme de complément.

Dans le cas d'une administration topique, la composition peut comprendre les constituants usuellement employés dans l'application envisagée.

On peut notamment citer l'eau, les solvants, les huiles d'origine minérale, animale et/ou végétale, les cires, les pigments, les charges, les tensioactifs, les actifs cosmétiques ou dermatologiques, les filtres UV, les polymères, les gélifiants, les conservateurs.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition selon l'invention peut se présenter sous toutes les formes galéniques normalement utilisées dans les domaines cosmétique et dermatologique; elle peut être notamment sous forme d'une solution aqueuse, hydroalcoolique, éventuellement gélifiée, d'une dispersion du type lotion éventuellement biphasée, d'une émulsion huile-dans-eau ou eau-dans-huile ou multiple, d'un gel aqueux, d'une dispersion d'huile dans une phase aqueuse à l'aide de sphérules, ces sphérules pouvant être des nanoparticules polymériques telles que les nanosphères et les nanocapsules ou des vésicules lipidiques de type ionique et/ou non-ionique.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les éventuels coémulsionnants utilisés dans la composition sous forme d'émulsion sont choisis parmi ceux classiquement utilisés dans le domaine considéré. L'émulsionnant et le coémulsionnant sont présents, dans la composition, en une proportion pouvant aller de 0,3 à 30 % en poids, et de préférence de 0,5 à 20 % en poids par rapport au poids total de la composition.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage.

Cette composition peut constituer une crème de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires); un fond de teint fluide, un lait de démaquillage, un lait corporel de protection ou de soin, un lait anti-solaire; une lotion, gel ou mousse pour le soin de la peau, comme une lotion de nettoyage.

Dans un aspect avantageux de l'invention, les compositions utilisées peuvent comporter en plus au moins un agent dépigmentant et/ou un agent desquamant, et/ou au moins un agent apaisant, et/ou au moins agent photoprotecteur organique et/ou au moins un agent photoprotecteur inorganique.

L'utilisation d'au moins un composé C-glycoside en association avec un autre agent dépigmentant pourra notamment permettre d'utiliser une quantité plus faible de chacun des dépigmentant.
Par agent « dépigmentant », on entend par exemple des agents dépigmentants ou anti-pigmentants les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.

En particulier, le C-glycoside pourra être associé dans une composition avec l'acide ascorbique (vitamine C) et/ou un de ses analogues ou dérivés.
Les analogues ou dérivés de l'acide ascorbique sont, plus particulièrement, ses sels, tels que notamment l'ascorbate de sodium, l'ascorbylphosphate de magnésium ou de sodium, ses esters, tels que notamment ses esters acétique, propionique ou palmitique, ou ses sucres, tels que notamment l'acide ascorbique glycosylé.
En raison de sa structure chimique (alpha-cétolactone) qui le rend très sensible à certains paramètres de l'environnement tels que la lumière, la chaleur et les milieux aqueux, il pourra être avantageux d'utiliser l'acide ascorbique sous la forme d'un ester d'ose de l'acide ascorbique ou d'un sel métallique d'acide ascorbique phosphorylé.
Les esters d'ose de l'acide ascorbique utilisables dans l'invention sont notamment les dérivés glycosylé, mannosylé, fructosylé, fucosylé, galactosylé N-acétylglucosaminé, N-acétylmuramique de l'acide ascorbique et leurs mélanges et plus spécialement l'ascorbyl-2 glucoside ou 2-O-α-D glucopyranosyl de l'acide L-ascorbique ou encore le 6-O-β-D galactopyranosyl de l'acide L-ascorbique. Ces derniers composés ainsi que leurs procédés de préparation, sont en particulier décrits dans les documents EP-A-487404, EP-A-425066 et J05213736.
De son côté, le sel métallique d'acide ascorbique phosphorylé est choisi parmi les ascorbyl phosphates de métal alcalin, les ascorbyl phosphates de métal alcalino-terreux et les ascorbyl phosphates de métal de transition. On utilise avantageusement l'ascorbyl phosphate de magnésium.

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;
- soit sur les enzymes impliquées dans la desquamation ou la dégradation des cornéodesmosomes, les glycosidases, la stratum comeum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP 0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme agents apaisants utilisables dans la composition selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3-stéaroyloxy glycyrrhetique), l'acide ursolique et ses sels, l'acide oléanolique et ses sels, l'acide bétulinique et ses sels, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique de vitamine E et C) de Seppic, les huiles insaturées en oméga 3 telles que les huiles de rosier musca, de cassis, d'ecchium, de poisson, des extraits de plancton, la capryloyl glycine, le Seppicalm VG (sodium palmitoylproline et nymphéa alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa moniera, les phytostérols, la cortisone, l'hydrocortisone, l'indométhacine et la beta méthasone.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans l'US 4,367,390, EP 0 863 145, EP 0 517 104, EP 0 570 838, EP 0 796 851, EP 0 775 698, EP 0 878 469, EP 0 933 376, EP 0 507 691, EP 0 507 692, EP 0 790 243, EP 0 944 624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP 0 669 323 et US 2,463,264; les dérivés de l'acide p-aminobenzoïque (PABA) ; les dérivés de méthylène bis-(hydroxyphényl benzotriazole) tels que décrits dans s les demandes US 5,237,071, US 5,166,355, GB 2303549, DE 197 26 184 et EP 0 893 119 ; et les polymères filtres et silicones filtres tels que ceux décrits notamment dans la demande WO-93/04665 ; les dimères dérivés d'α-alkylstyrène tels que ceux décrits dans la demande de brevet DE 198 55 649.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP 0 518 772 et EP 0 518 773.

Les agents photoprotecteurs sont généralement présents dans la composition selon l'invention dans des proportions allant de 0,1 à 20 % en poids par rapport au poids total de la composition, et de préférence allant de 0,2 à 15 % en poids par rapport au poids total de la composition.

De préférence, le ou les dérivés C-glycosides seront associés avec au moins un agent dépigmentant et/ou au moins un agent photoprotecteur.

Les exemples qui suivent illustrent l'invention sans en limiter la portée. Les composés sont, selon le cas, cités en noms chimiques ou en noms CTFA (International Cosmetic Ingredient Dictionary and Handbook).

### Exemple 1 : mise en évidence de l'activité dépigmentante du C-β-D-xylopyranoside-n-propane-2-one

Une étude clinique monocentrique, comparative (actif contre placebo constitué du véhicule de l'actif, voir la formule détaillée ci-dessous) en double aveugle randomisée sur sa localisation (le sujet étant son propre témoin) a été conduite chez 15 femmes âgées en moyenne de 62 ans, de phototype III (sur une échelle de 6 niveaux : I - brûle toujours, ne bronze jamais ; II - brûle toujours, bronze peu ; III - brûle modérément, bronze progressivement ; IV - brûle faiblement, bronze très facilement ; V - brûle rarement, bronze intensément ; VI - ne brûle jamais, fortement pigmenté) présentant des signes de vieillissement global du visage modérés.
Une évaluation clinique des lentigines actiniques a été effectuée par un dermatologue en début et en fin d'étude selon une échelle à 7 niveaux dont 4 niveaux principaux :
o 0 - absent ;
o 1 - taches peu nombreuses ;
o 2 - taches nombreuses ;
o 3 - taches très nombreuses
et 3 niveaux intermédiaires (0,5 ; 1,5 ; 2,5).
Les femmes ont appliqué deux fois par jour pendant 3 mois (98 jours) sur les zones prétagiennes le C-β-D-xytopyranoside-n-propane-2-one à une concentration de 10% en poids par rapport au poids total de la composition à raison de 2 mg/cm² ou le placebo.

### Composition de l'excipient :

| **Nom Chimique** | |
|---|---|
| VASELINE BLANCHE | 4 |
| TRI-STEARATE DE SORBITANE | 0.9 |
| STEARATE DE POLYETHYLENE GLYCOL (40 OE) | 2 |
| PARFUM | 0.25 |
| MYRISTATE DE MYRISTYLE | 2 |
| MELANGE P-HYDROXYBENZOATES DE METHYL, BUTYLE, ETHYLE, PROPYLE, ISOBUTYLE (7/57/22/14) | 0.3 |
| MELANGE MONO/DISTEARATE (36/64) DE GLYCERYLE/STEARATE DE POTASSIUM | 3 |
| ISOPARAFFINE (6-8 MOLES D'ISOBUTYLENE) HYDROGENEE | 8.5 |
| HYDROXYDE DE SODIUM PUR | 0.05 |
| GLYCEROL | 3 |
| EAU DESIONISEE MICROBIOLOGIQUEMENT PROPRE | 35.84 |
| DIGLUCONATE DE CHLORHEXIDINE EN SOLUTION | 0.25 |
| CYCLOPENTA DIMETHYLSILOXANE | 5 |
| ALCOOL CETYLIQUE PUR BIDISTILLE | 4 |
| ACIDE GRAS (ACIDE STEARIQUE MAJORITAIRE) D'ORIGINE VEGETALE | 1.2 |
| ACIDE ETHYLENE DIAMINE TETRACETIQUE, SEL DISODIQUE, 2 H20 | 0.15 |
| ACETATE DE DL-ALPHA-TOCOPHERYLE (ACETATE DE VITAMINE E) | 0.3 |
| 4-METHOXYCINNAMATE DE 2-ETHYL HEXYLE PROTEGE | 0.52 |
| 1.5-ANHYDRO-6.8-DIDEOXY-L-GLUCO-OCTITOL A 30% DANS L'EAU | 28.74 |

Le score moyen du coté traité par le C-β-D-xylopyranoside-n-propane-2-one diminue de 1,07+/-0,62 avant traitement à 0,93+/-0,68 (p=0,08) alors que du coté placebo, ce score augmente de 1,10+/-0,69 à 1,13+/-0,67 (NS).
Ainsi, 54% des sujets présentent un score moins sévère du coté traité contre 13% en début d'étude.

### Exemple 2 : exemple de composition topique

On prépare une crème blanchissante de soin du visage de type émulsion huile-dans-eau, comprenant (% en poids) :
- C-β-D-xylopyranoside-n-propane-2-one 0,005%
- stéarate de glycérol 2%
- polysorbate 60 (Tween 60 de ICI) 1%
- acide stéarique 1,4%
- triéthanolamine 0,7%
- carbomer 0,4%
- fraction liquide du beurre de karité 12%
- perhydrosqualène 12%
- antioxydant 0,05%
- parfum, conservateur qs
- eau qsp 100%

### Exemple 3 : exemple de composition topique

On prépare un gel dépigmentant pour la peau comprenant (% en poids) :
- C-β-D-xylopyranoside-n-propane-2-one 2%
- hydroxypropylcellulose (Klucel H de Hercules) 1%
- antioxydant 0,05%
- isopropanol 40%
- parfum, conservateur qs
- eau qsp 100%

### Exemple 4 : exemple de composition orale

On prépare des dragées comprenant (% en poids) :

### Matières actives mg/dragée

| | |
|---|---|
| C-β-D-xylopyranoside-n-propane-2-one | 300 |

### Excipient du noyau de la dragée

| | |
|---|---|
| Cellulose mino-cristalline | 70 |
| Encompress^{™} | 60 |
| Stearate de Magnesium | 3 |
| Silice colloïdale anhydre | 1 |

### Agent d'enrobage

| | |
|---|---|
| Gomme laque | 5 |
| Talc | 61 |
| Polyvidone | 6 |

Ce type de dragée peut être pris 1 à 4 fois par jour.

## Revendications

1. Utilisation non-thérapeutique d'au moins un dérivé C-glycoside répondant à la formule (I) suivante : 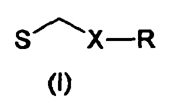 dans laquelle,
- R désigne un radical linéaire en C₁-C₄, éventuellement substitué par -OH, -COOH ou - COOR"₂, R"₂ étant un radical alkyle saturé en C₁-C₄,
- X représente un radical choisi parmi les groupements : 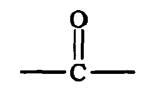 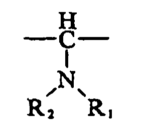 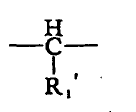 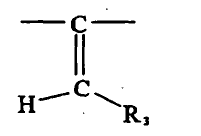 avec R₁, R₂ et R₃ représentant, indépendamment l'un de l'autre, un atome d'hydrogène, ou un radical R, avec R tel que défini précédemment, et R'₁ représente un atome d'hydrogène, un groupe -OH ou un radical R, avec R tel que défini précédemment, R1 pouvant désigner également un radical aryle en C₆ à C₁₀
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être a ou β, ainsi que leurs sels cosmétiquement acceptables, leurs solvates tels que les hydrates, en tant qu'agent blanchissant de la peau et/ou comme agent anti-brunissement.

2. Utilisation selon la revendication 1, **caractérisée en ce que** :
- R désigne un radical linéaire en C₁-C₄, éventuellement substitué par -OH, -COOH ou - COOR"₂, R"₂ étant un radical alkyle saturé en C₁-C₄,
- S représente un monosaccharide comme décrit dans la revendication 1 ; et
- X représente une groupement choisi parmi -CO-, -CH(OH)-, -CH(NH₂)-, -CH(NHCH₂CH₂CH₂OH)-, -CH(NHPh)-, -CH(CH₃)-.

3. Utilisation selon la revendication 2, **caractérisée en ce que** R désigne un radical linéaire en C₁-C₃, éventuellement substitué par -OH, -COOH ou -COOR"₂, R"₂ étant un radical alkyle saturé en C₁-C₄.

4. Utilisation selon la revendication 2 ou 3, **caractérisée en ce que** X représente une groupement choisi parmi -CO-, -CH(OH)-, -CH(NH₂)-.

5. Utilisation selon l'une quelconque des revendications précédentes, **caractérisée en ce que** S représente un monosaccharide choisi parmi le D-glucose, le D-xylose, la N-acétyl-D-glucosamine ou le L-fucose, et en particulier le D-xylose.

6. Utilisation selon l'une quelconque des revendications précédentes, **caractérisée en ce que** R désigne un groupe méthyle.

7. Utilisation selon l'une quelconque des revendications précédentes pour éclaircir et/ou blanchir et/ou uniformiser la couleur d'une peau brunie, prévenir la formation et/ou éliminer les taches pigmentaires brunâtres et/ou les taches de sénescence et/ou les éphélides.

8. Utilisation d'au moins un dérivé C-glycoside répondant à la formule (I) suivante : 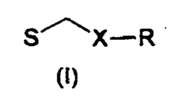 dans laquelle,
- R désigne un radical linéaire en C₁-C₄, éventuellement substitué par -OH, -COOH ou - COOR"₂, R"₂ étant un radical alkyle saturé en C₁-C₄,
- X représente un radical choisi parmi les groupements : 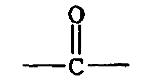 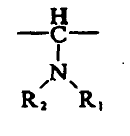 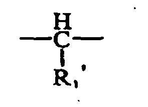 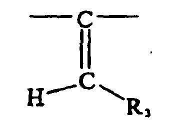 avec R₁, R₂ et R₃ représentant, indépendamment l'un de l'autre, un atome d'hydrogène, ou un radical R, avec R tel que défini précédemment, et R'₁, représente un atome d'hydrogène, un groupe -OH ou un radical R, avec R tel que défini précédemment, R1 pouvant désigner également un radical aryle en C₆ à C₁₀
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être α ou β, ainsi que leurs sels cosmétiquement acceptables, leurs solvates tels que les hydrates, pour la préparation d'une composition destinée au traitement de désordres de la pigmentation.

9. Utilisation selon la revendication 8, **caractérisée en ce que** :
- R désigne un radical linéaire en C₁-C₄, éventuellement substitué par -OH, -COOH ou - COOR"₂, R"₂ étant un radical alkyle saturé en C₁-C₄,
- S représente un monosaccharide comme décrit dans la revendication 8: et
- X représente une groupement choisi parmi -CO-, -CH(OH)-, -CH(NH₂)-. -CH(NHCH₂CH₂CH₂OH)-, -CH(NHPh)-, -CH(CH₃)-.

10. Utilisation selon la revendication 9, **caractérisée en ce que** R désigne un radical **II-**néaire en C₁-C₃, éventuellement substitué par -OH, -COOH ou -COOR"₂, R"₂ étant un radical alkyle saturé en C₁-C₄.

11. Utilisation selon la revendication 9 ou 10, **caractérisée en ce que** X représente une groupement choisi parmi -CO-, -CH(OH)-, -CH(NH₂)-.

12. Utilisation selon l'une quelconque des revendications 8 à 11, **caractérisée en ce que** S représente un monosaccharide choisi parmi le D-glucose, le D-xylose, la N-acétyl-D-glucosamine ou le L-fucose, et en particulier le D-xylose.

13. Utilisation selon l'une quelconque des revendications 8 à 12, **caractérisée en ce que** R désigne un groupe méthyle.

14. Utilisation selon l'une quelconque des revendications 8 à 13, **caractérisée en ce que** la composition est destinée à traiter le mélasma des avant-bras, les mélasmas idiopathiques, les hyperpigmentations associées à la grossesse ou à une contraception oestro-progestative, les puva-lentigines, les hyperpigmentations accidentelles, les hyperpigmentations dues à des leucodermies, le vitiligo.

15. Utilisation selon l'une quelconque des revendications précédentes, **caractérisée en ce que** ledit dérivé est choisi parmi :
1. C-β-D-xylopyranoside-n-propane-2-one ;
2. C-α-D-xylopyranoside-n-propane-2-one ;
3. 1-[2-(3-hydroxy-propylamino)-propyl]- C-β-D-xylopyranose ;
4. 1-[2-(3-hydroxy-propylamino)propyl]-C-α-D-xylopyranose ;
5. C-β-D-xylopyranoside-2-hydroxy-propane ;
6. C-α-D-xylopyranoside-2-hydroxy-propane ;
7. C-β-D-xylopyranoside-2-amino-propane ;
8. C-α-D-xylopyranoside-2-amino-propane ;
9. C-β-D-xylopyranoside-2-phénylamino-propane ;
10. C-α-D-xylopyranoside-2-phénylamino-propane ;
11. ester éthylique de l'acide 3-méthyl-4-(C-β-D-xylopyranoside)-butyrique ;
12. ester éthylique de l'acide 3-méthyl-4-(C-α-D-xylopyranoside)-butyrique ;
13. acide 6-(C-β-D-xylopyranoside)-5-céto-hexanoique ;
14. acide 6-(C-α-D-xylopyranoside)-5-céto-hexanoique ;
15. acide 6-(C-β-D-xylopyranoside)-5-hydroxy-hexanoique ;
16. acide 6-(C-α-D-xylopyranoside)-5-hydroxy-hexanoique ;
17. acide 6-(C-β-D-xylopyranoside)-5-amino-hexanoique ;
18. acide 6-(C-α-D-xylopyranoside)-5-amino-hexanoique
19. acide 6-(C-β-D-xylopyranoside)-5-phenylamino-hexanoique ;
20. acide 6-(C-α-D-xylopyranoside)-5-phenylamino-hexanoique ;
21. 1-(C-β-D-xylopyranoside)hexane-2,6-diol ;
22. 1-(C-α-D-xylopyranoside)-hexane-2,6-diol ;
23. acide 5-(C-β-D-xylopyranoside)-4-céto-pentanoique ;
24. acide 5-(C-α-D-xylopyranoside)-4-céto-pentanoique ;
25. acide 5-(C-β-D-xylopyranoside)-4-hydroxy-pentanoique ;
26. acide 5-(C-α-D-xylopyranoside)-4-hydroxy-pentanoique ;
27. acide 5-(C-β-D-xylopyranoside)4-amino-pentanoique ;
28. acide 5-(C-α-D-xylopyranoside)-4-amino-pentanoique ;
29. acide 5-(C-β-D-xylopyranoside)-4-phénylamino-pentanoique ;
30. acide 5-(C-α-D-xylopyranoside)-4-phénylamino-pentanoique ;
31. 1-(C-β-D-xylopyranoside)-pentane-2,5-diol ;
32. 1-(C-α-D-xylopyranoside)-pentane-2,5-diol
33. 1-(C-β-D-fucopyranoside)-propane-2-one ;
34. 1-(C-α-D-fucopyranoside)-propane-2-one ;
35. 1-(C-β-L-fucopyranoside)-propane-2-one ;
36. 1-(C-α-L-fucopyranoside)-propane-2-one;
37. 1-(C-β-D-fucopyranoside)-2-hydroxy-propane ;
38. 1-(C-α-D-fucopyranoside)-2-hydroxy-propane ;
39. 1-(C-β-L-fucopyranoside)-2-hydroxy-propane ;
40. 1-(C-α-L-fucopyranoside)-2-hydroxy-propane ;
41. 1-(C-β-D-fucopyranoside)-2-amino-propane ;
42. 1-(C-α-D-fucopyranoside)-2-amino-propane ;
43. 1-(C-β-L-fucopyranoside)-2-amino-propane ;
44. 1-(C-α-L-fucopyranoside)-2-amino-propane ;
45. 1-(C-β-D-fucopyranoside)-2-phénylamino-propane ;
46. 1-(C-α-D-fucopyranoside)2-phénylamino-propane ;
47. 1-(C-β-L-fucopyranoside)-2-phénylamino-propane ;
48. 1-(C-α-L-fucopyranoside)-2-phénylamino-propane ;
49. ester éthylique de l'acide 3-méthyl-4-(C-β-D-fucopyranoside)-butyrique ;
50. ester éthylique de l'acide 3-méthyl-4-(C-α-D-fucopyranoside)-butyrique ;
51. ester éthylique de l'acide 3-méthyl-4-(C-β-L-fucopyranoside)-butyrique ;
52. ester éthylique de l'acide 3-méthyl-4-(C-α-L-fucopyranoside)-butyrique ;
53. acide 6-(C-β-D-fucopyranoside)-5-céto-hexanoique ;
54. acide 6-(C-α-D-fucopyranoside)-5-céto-hexanoique ;
55. acide 6-(C-β-L-fucopyranoside)-5-céto-hexanoique ;
56. acide 6-(C-α-L-fucopyranoside)-5-céto-hexanoique ;
57. acide 6-(C-β-D-fucopyranoside)-5-hydroxy-hexanoique ;
58. acide 6-(C-α-D-fucopyranoside)-5-hydroxy-hexanoique ;
59. acide 6-(C-β-L-fucopyranoside)-5-hydroxy-hexanoique ;
60. acide 6-(C-α-L-fucopyranoside)-5-hydroxy-hexanoique ;
61. acide 6-(C-β-D-fucopyranoside)-5-amino-hexanoique ;
62. acide 6-(C-α-D-fucopyranoside)-5-amino-hexanoique ;
63. acide 6-(C-β-L-fucopyranoside)-5-amino-hexanoique ;
64. acide 6-(C-α-L-fucopyranoside)-5-amino-hexanoique ;
65. 1-(C-β-D-fucopyrranoside)-hexane-2,6-diol ;
66. 1-(C-α-D-fucopyranoside)-hexane-2,6-diol ;
67. 1-(C-β-L-fucopyranoside)-hexane-2,6-diol ;
68. 1-(C-α-L-fucopyranoside)-hexane-2,6-diol ;
69. acide 5-(C-β-D-fucopyranoside)-4-céto-pentanoique ;
70. acide 5-(C-α-D-fucopyranoside)-4-céto-pentanoique ;
71. acide 5-(C-β-L-fucopyranoside)-hexane-2,6-diol)-4-céto-pentanoique ;
72. acide 5-(C-α-L-fucopyranoside)-hexane-2,6-diol)-4-céto-pentanoique ;
73. acide 5-(C-β-D-fucopyranoside)-4-hydroxy-pentanoique ;
74. acide 5-(C-α-D-fucopyranoside)-4-hydroxy-pentanoique ;
75. acide 5-(C-β-L-fucopyranoside)-4-hydroxy-pentanoique ;
76. acide 5-(C-α-L-fucopyranoside)-4-hydroxy-pentanoique ;
77. acide 5-(C-β-D-fucopyranoside)-4-amino-pentanoique ;
78. acide 5-(C-α-D-fucopyranoside)-4-amino-pentanoique
79. acide 5-(C-β-L-fucopyranoside)-4-amino-pentanoique ;
80. acide 5-(C-α-L-fucopyranoside)-4-amino-pentanoique ;
81. 1-(C-β-D-fucopyranoside)-pentane-2,5-diol ;
82. 1-(C-α-D-fucopyranoside)-pentane-2,5-diol ;
83. 1-(C-β-L-fucopyranoside)-pentane-2,5-diol ;
84. 1-(C-α-L-fucopyranoside)-pentane-2,5-diol ;
85. 1-(C-β-D-Glucopyranosyl)-2-hydroxyl-propane ;
86. 1-(C-α-D-Glucopyranosyl)-2-hydroxyl-propane ;
87. 1-(C-β-D-Glucopyranosyl)-2-amino-propane ;
88. 1-(C-α-D-Glucopyranosyl)-2-amino-propane ;
89. 1-(C-β-D-Glucopyranosyl)-2-phénylamino-propane ;
90. 1-(C-α-D-Glucopyranosyl)-2-phénylamino-propane ;
91. ester éthylique de l'acide 3-méthyl-4-(C-β-D-Glucopyranosyl)-butyrique ;
92. ester éthylique de l'acide 3-méthyl-4-(C-α-D-Glucopyranosyl)-butyrique ;
93. acide 6-(C-β-D-Glucopyranosyl)-5-céto-hexanoique ;
94. acide 6-(C-α-D-Glucopyranosyl)-5-céto-hexanoique ;
95. acide 6-(C-β-D-Glucopyranosyl)-5-hydroxy-hexanoique ;
96. acide 6-(C-α-D-Glucopyranosyl)-5-hydroxy-hexanoique ;
97. acide 6-(C-β-D-Glucopyranosyl)-5-amino-hexanoique ;
98. acide 6-(C-α-D-Glucopyranosyl)-5-amino-hexanoique ;
99. acide 6-(C-β-D-Glucopyranosyl)-5-phénylamino-hexanoique ;
100. acide 6-(C-α-D-Glucopyranosyl)-5-phénylamino-hexanoique ;
101. 1-(C-β-D-Glucopyranosyl)hexane-2,6-diol ;
102. 1-(C-α-D-Glucopyranosyl)-hexane-2,6-diol ;
103. acide 6-(C-β-D-Glucopyranosy)-5-céto-pentanoique ;
104. acide 6-(C-α-D-Glucopyranosyl)-5-céto-pentanoique ;
105. acide 6-(C-β-D-Glucopyranosyl)-5-hydroxy-pentanoique ;
106. acide 6-(C-α-D-Glucopyranosyl)-5-hydroxy-pentanoique ;
107. acide 6-(C-β-D-Glucopyranosyl)-5-amino-pentanoique ;
108. acide 6-(C-α-D-Glucopyranosyl)-5-hydroxy-pentanoique ;
109. acide 6-(C-β-D-Glucopyranosyl)-5-phénylamino-pentanoique ;
110. acide 6-(C-α-D-Glucopyranosyl)-5-phénylamino-pentanoique ;
111. 1-(C-β-D-Glucopyranosyl)-pentane-2,5-diol ;
112. 1-(C-α-D-Glucopyranosyl)-pentane-2,5-diol ;
113. 1-(C-β-D-galactopyranosyl)-2-hydroxyl-propane ;
114. 1-(C-α-D-galactopyranosyl)-2-hydroxyl-propane ;
115. 1-(C-β-D-galactopyranosyl)-2-amino-propane ;
116. 1-(C-α-D-galactopyranosyl)-2-amino-propane :
117. 1-(C-β-D-galactopyranosyl)-2-phénylamino-propane ;
118. 1-(C-α-D-galactopyranosyl-2-phénylamino-propane ;
119. ester éthylique de l'acide 3-méthyl-4-(C-β-D-galactopyranosyl)-butyrique ;
120. ester éthylique de l'acide 3-méthyl-4-(C-α-D-galactopyranosyl)-butyrique ;
121. acide 6(C-β-D-galactopyranosyl)-5-céto-hexanoique ;
122. acide 6-(C-α-D-galactopyranosyl)-5-céto-hexanoique ;
123. acide 6-(C-β-D-galactopyranosyl)-5-hydroxy-hexanoique ;
124. acide 6-(C-α-D-galactopyranosyl)5-hydroxy-hexanoique ;
125. acide 6-(C-β-D-galactopyranosyl)-5-amino-hexanoique
126. acide 6-(C-α-D-galactopyranosyl)-5-amino-hexanoique ;
127. acide 8-(C-β-D-galactopyranosyl)5-phénylamino-hexanoique ;
128. acide 6-(C-α-D-galactopyranosyl)5-phénylamino-hexanoique ;
129. 1-(C-β-D-galactopyranosyl)-hexane-2,6-diol ;
130. 1-(C-α-D-galactopyranosyl)-hexane-2,6-diol ;
131. acide 6-(C-β-D-galactopyranosyl)-5-céto-pentanoique ;
132. acide 6-(C-α-D-galactopyranosyl)-5-céto-pentanoique ;
133. acide 6-(C-β-D-galactopyranosyl)-5-hydroxy-pentanoique ;
134. acide 6-(C-α-D-galactopyranosyl)-5-hydroxy-pentanoique ;
135. acide 6-(C-β-D-galactopyranosyl)-5-amino-pentanoique ;
136. acide 6-(C-α-D-galactopyranosyl)-5-amino-pentanoique ;
137. acide 6-(C-β-D-galactopyranosyl)-5-phénylamino-pentanoique ;
138. acide 6-(C-α-D-galactopyranosyl)-5-phènylamino-pentanoique ;
129. 1-(C-β-D-galactopyranosyl)-pentane-2,6-diol ;
140. 1-(C-α-D-galactopyranosyl)-pentane-2,6-diol ;
141. 1-(C-β-D-fucoturanosyl)-propane-2-one ;
142. 1-(C-α-D-fucofuranosyl)-propane-2-one ;
143. 1-(C-β-L-fucofuranosyl)-propane-2-one ;
144. 1-(C-α-L-fucofuranosyl)propane-2-one ;
145. 3'-(acétamido-C-β-D-glucopyranosyl)-propane-2'-one ;
146. 3'-(acétamido-C-α-D-glucopyranosyl)-propane-2'-one ;
147. 1-(C-β-D-galactopyranosyl)-2-hydroxyl-propane ;
148. 1-(C-α-D-galactopyranosyl)-2-amino-propane ;
149. 1-(acétamido-C-β-D-glucopyranosyl)-2-phénylamino-propane ;
150. 1-(acétamido-C-α-D-glucopyranosyl)-2-phénylamino-propane ;
151. ester éthylique de l'acide 3-méthyl-4-(acétamido-C-β-D-glucopyranosyl)-butyrique ;
152. ester éthylique de l'acide 3-méthyl-4-(acétamido-C-α-D-glucopyranosyl)-butyrique ;
153. acide 6-(acétamido-C-β-D-glucopyranosyl)-5-céto-hexanoique;
154. acide 6-(acétamido-C-α-D-glucopyranosyl)-5-céto-hexanoique ;
155. acide 6-(acétamido-C-β-D-glucopyranosyl)-5-hydroxy-hexanoique ;
156. acide 6-(acétamido-C-α-D-glucopyranosyl)-5-hydroxy-hexanoique ;
157. acide 6-(acétamido-C-β-D-glucopyranosyl)-5-amino-hexanoique ;
158. acide 6-(acétamido-C-α-D-glucopyranosyl)-5-amino-hexanoique ;
159. acide 6-(acétamido-C-β-D-glucopyranosyl)5-phénylamido-hexanoique :
160. acide 6-(acétamido-C-α-D-glucopyranosyl-5-phénylamino-hexanoique ;
161. 1-(acétamido-C-β-D-glucopyranosyl)-hexane-2,6-diol ;
162. 1-(acétamido-C-α-D-glucopyranosyl)-hexane-2,6-diol ;
163. acide 6-(acétamido-C-β-D-glucopyranosyl)-5-céto-pentanoique ;
164. acide 6-(acétamido-C-α-D-glucopyranosyl)-céto-pentanoique ;
165. acide 6-(acétamido-C-β-D-glucopyranosyl)-5-hydroxy-pentanoique ;
166. acide 6-(acétamido-C-α-D-glucopyranosyl)-5-hydroxy-pentanoique ;
167. acide 6-(acétamido-C-β-D-glucopyranosyl)-5-amino-pentanoique ;
168. acide 6-(acétamido-C-α-D-glucopyranosyl)-5-amino-pentanoique ;
169. acide 6-(acétamido-C-β-D-glucopyranosyl)-5-phénylamino-pentanoique ;
170. acide 6-acétamido-C-α-D-glucopyranosyl)-5-phénylamino-pentanoique ;
171. 1-(acétamido-C-β-D-glucopyranosyl)-pentane-2,5-diol ;
172. 1-(acétamido-C-α-D-glucopyranosyl)-pentane-2,5-diol.

16. Utilisation selon l'une quelconque des revendications 8 à 15, **caractérisée en ce que** ledit dérivé est utilisé en une quantité représentant de 0,0001% à 25% du poids total de la composition.

17. Utilisation selon la revendication 16, **caractérisée en ce que** ledit dérivé est utilisé en une quantité représentant de 0,001% à 10% du poids total de la composition.

18. Utilisation selon l'une quelconque des revendications 8-17, **caractérisée en ce que** ledit dérivé C-glycoside est associé avec au moins un agent dépigmentant et/ou au moins agent photoprotecteur organique et/ou au moins un agent photoprotecteur inorganique.

19. Procédé non-thérapeutique cosmétique de blanchiment de la peau et/ou du cuir chevelu et/ou des muqueuses comprenant l'ingestion ou l'application sur la peau et/ou du cuir chevelu et/ou des muqueuses d'au moins un composé C-glycoside répondant à la formule (I) tel que défini dans l'une quelconque des revendications précédentes.

## Claims

1. Non-therapeutic use of at least one C-glycoside derivative corresponding to the following formula (I): 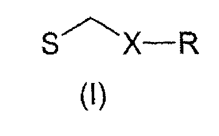 in which:
- R denotes a linear C₁-C₄ radical optionally substituted by -OH, -COOH or -COOR"₂, R"₂ being a saturated C₁-C₄ alkyl radical,
- X represents a radical chosen from the groups: 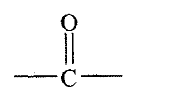 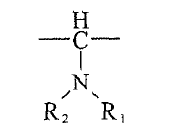 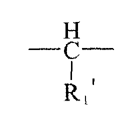 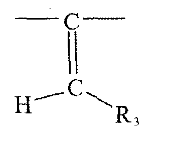 with R₁, R₂ and R₃ representing, independently of one another, a hydrogen atom or a radical R, with R as defined above, and R'₁ represents a hydrogen atom, an -OH group or a radical R, with R as defined above, it being possible for R₁ also to denote a C₆ to C₁₀ aryl radical;
- S represents a monosaccharide or a poly-saccharide comprising up to 20 sugar units, in particular up to 6 sugar units, in a pyranose and/or furanose form and of the L and/or D series, it being possible for the said mono- or polysaccharide to be substituted by a necessarily free hydroxyl group and optionally one or more optionally protected amine functional group(s), and
- the S-CH₂-X bond represents a bond of C-anomeric nature which can be α or β,
and their cosmetically acceptable salts, their solvates as well as the hydrates,
as whitening agent for the skin and/or as antibrowning agent.

2. Use according to Claim 1, **characterized in that**:
- R denotes a linear C₁-C₄ radical optionally substituted by -OH, -COOH or -COOR"₂, R"₂ being a saturated C₁-C₄ alkyl radical,
- S represents a monosaccharide as described in Claim 1, and
- X represents a group chosen from -CO-, -CH(OH)-,
- CH(NH₂)-, -CH(NHCH₂CH₂CH₂OH)-, -CH(NHPh)- or -CH(CH₃)-.

3. Use according to Claim 2, **characterized in that** R denotes a linear C₁-C₃ radical optionally substituted by -OH, -COOH or -COOR"₂, R"₂ being a saturated C₁-C₄ alkyl radical.

4. Use according to Claim 2 or 3, **characterized in that** X represents a group chosen from -CO-, -CH(OH)- or -CH(NH₂)-.

5. Use according to any one of the preceding claims, **characterized in that** S represents a monosaccharide chosen from D-glucose, D-xylose, N-acetyl-D-glucosamine or L-fucose and in particular D-xylose.

6. Use according to any one of the preceding claims, **characterized in that** R denotes a methyl group.

7. Use according to any one of the preceding claims for lightening and/or whitening and/or rendering uniform the colour of a browned skin, preventing the formation of and/or removing brownish pigment blemishes and/or blemishes due to ageing and/or freckles.

8. Use of at least one C-glycoside derivative corresponding to the following formula (I): 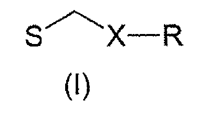 in which:
- R denotes a linear C₁-C₄ radical optionally substituted by -OH, -COOH or -COOR"₂, R"₂ being a saturated C₁-C₄ alkyl radical,
- X represents a radical chosen from the groups: 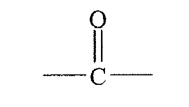 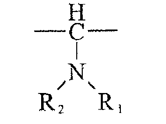 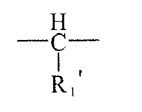 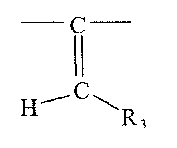 with R₁, R₂ and R₃ representing, independently of one another, a hydrogen atom or a radical R, with R as defined above, and R'₁ represents a hydrogen atom, an -OH group or a radical R, with R as defined above, it being possible for R₁ also to denote a C₆ to C₁₀ aryl radical;
- S represents a monosaccharide or a poly-saccharide comprising up to 20 sugar units, in particular up to 6 sugar units, in a pyranose and/or furanose form and of the L and/or D series, it being possible for the said mono- or polysaccharide to be substituted by a necessarily free hydroxyl group and optionally one or more optionally protected amine functional group(s), and
- the S-CH₂-X bond represents a bond of C-anomeric nature which can be α, or β, and their cosmetically acceptable salts, their solvates as well as the hydrates,
in the preparation of a composition intended for the treatment of disorders of pigmentation.

9. Use according to Claim 8, **characterized in that**:
- R denotes a linear C₁-C₄ radical optionally substituted by -OH, -COOH or -COOR"₂, R"₂ being a saturated C₁-C₄ alkyl radical,
- S represents a monosaccharide as described in Claim 8, and
- X represents a group chosen from -CO-, -CH(OH)-, -CH(NH2)-, -CH(NHCH₂CH₂CH₂OH)-, -CH (NHPh) - or -CH(CH₃)-.

10. Use according to Claim 9, **characterized in that** R denotes a linear C₁-C₃ radical optionally substituted by -OH, -COOH or -COOR"₂, R"₂ being a saturated C₁-C₄ alkyl radical.

11. Use according to Claim 9 or 10, **characterized in that** X represents a group chosen from -CO-, -CH(OH)- or -CH(NH₂)-.

12. Use according to any one of Claims 8 to 11, **characterized in that** S represents a monosaccharide chosen from D-glucose, D-xylose, N-acetyl-D-glucosamine or L-fucose and in particular D-xylose.

13. Use according to any one of Claims 8 to 12, **characterized in that** R denotes a methyl group.

14. Use according to any one of Claims 8 to 13, **characterized in that** the composition is intended to treat melasma of the forearms, idiopathic melasmas, hyperpigmentations associated with pregnancy or with oestrone-progestogen contraception, PUVA lentigines, accidental hyperpigmentations, hyperpigmentations due to leucodermas or vitiligo.

15. Use according to any one of the preceding claims, **characterized in that** the said derivative is chosen from:
1. C-β-D-xylopyranoside-n-propan-2-one;
2. C-α-D-xylopyranoside-n-propan-2-one;
3. 1-[2-(3-hydroxypropylamino)propyl]-C-β-D-xylopyranose;
4. 1-[2-(3-hydroxypropylamino)propyl]-C-α-D-xylopyranose;
5. C-β-D-xylopyranoside-2-hydroxypropane;
6. C-α-D-xylopyranoside-2-hydroxypropane;
7. C-β-D-xylopyranoside-2-aminopropane;
8. C-α-D-xylopyranoside-2-aminopropane;
9. C-β-D-xylopyranoside-2-(phenylamino)propane;
10. C-α-D-xylopyranoside-2-(phenylamino)propane;
11. ethyl ester of 3-methyl-4-(C-β-D-xylopyranoside)butyric acid;
12. ethyl ester of 3-methyl-4-(C-α-D-xylopyranoside)butyric acid;
13. 6-(C-β-D-xylopyranoside)-5-ketohexanoic acid;
14. 6-(C-α-D-xylopyranoside)-5-ketohexanoic acid;
15. 6-(C-β-D-xylopyranoside)-5-hydroxyhexanoic acid;
16. 6-(C-α-D-xylopyranoside)-5-hydroxyhexanoic acid;
17. 6-(C-β-D-xylopyranoside)-5-aminohexanoic acid;
18. 6-(C-α-D-xylopyranoside)-5-aminohexanoic acid;
19. 6-(C-β-D-xylopyranoside)-5-(phenylamino)hexanoic acid;
20. 6-(C-α-D-xylopyranoside)-5-(phenylamino)hexanoic acid;
21. 1-(C-β-D-xylopyranoside)hexane-2,6-diol;
22. 1-(C-α-D-xylopyranoside)hexane-2,6-diol;
23. 5-(C-β-D-xylopyranoside)-4-ketopentanoic acid;
24. 5-(C-α-D-xylopyranoside)-4-ketopentanoic acid;
25. 5-(C-β-D-xylopyranoside)-4-hydroxypentanoic acid;
26. 5-(C-α-D-xylopyranoside)-4-hydroxypentanoic acid;
27. 5-(C-β-D-xylopyranoside)-4-aminopentanoic acid;
28. 5-(C-α-D-xylopyranoside)-4-aminopentanoic acid;
29. 5-(C-β-D-xylopyranoside)-4-(phenylamino)pentanoic acid;
30. 5-(C-α-D-xylopyranoside)-4-(phenylamino)pentanoic acid;
31. 1-(C-β-D-xylopyranoside)pentane-2,5-diol;
32. 1-(C-α-D-xylopyranoside)pentane-2,5-diol;
33. 1-(C-β-D-fucopyranoside)propan-2-one;
34. 1-(C-α-D-fucopyranoside)propan-2-one;
35. 1-(C-β-L-fucopyranoside)propan-2-one;
36. 1-(C-α-L-fucopyranoside)propan-2-one;
37. 1-(C-β-D-fucopyranoside)-2-hydroxypropane;
38. 1-(C-α-D-fucopyranoside)-2-hydroxypropane;
39. 1-(C-β-L-fucopyranoside)-2-hydroxypropane;
40. 1-(C-α-L-fucopyranoside)-2-hydroxypropane;
41. 1-(C-β-D-fucopyranoside)-2-aminopropane;
42. 1-(C-α-D-fucopyranoside)-2-aminopropane;
43. 1-(C-β-L-fucopyranoside)-2-aminopropane;
44. 1-(C-α-L-fucopyranoside)-2-aminopropane;
45. 1-(C-β-D-fucopyranoside)-2-(phenylamino)propane;
46. 1-(C-α-D-fucopyranoside)-2-(phenylamino)propane;
47. 1-(C-β-L-fucopyranoside)-2-(phenylamino)propane;
48. 1-(C-α-L-fucopyranoside)-2-(phenylamino)propane;
49. ethyl ester of 3-methyl-4-(C-β-D-fucopyranoside)butyric acid;
50. ethyl ester of 3-methyl-4-(C-α-D-fucopyranoside)butyric acid;
51. ethyl ester of 3-methyl-4-(C-β-L-fucopyranoside)butyric acid;
52. ethyl ester of 3-methyl-4-(C-α-L-fucopyranoside)butyric acid;
53. 6-(C-β-D-fucopyranoside)-5-ketohexanoic acid;
54. 6-(C-α-D-fucopyranoside)-5-ketohexanoic acid;
55. 6-(C-β-L-fucopyranoside)-5-ketohexanoic acid;
56. 6-(C-α-L-fucopyranoside)-5-ketohexanoic acid;
57. 6-(C-α-D-fucopyranoside)-5-hydroxyhexanoic acid;
58. 6-(C-α-D-fucopyranoside)-5-hydroxyhexanoic acid;
59. 6-(C-β-L-fucopyranoside)-5-hydroxyhexanoic acid;
60. 6-(C-α-L-fucopyranoside)-5-hydroxyhexanoic acid;
61. 6-(C-β-D-fucopyranoside)-5-aminohexanoic acid;
62. 6-(C-α-D-fucopyranoside)-5-aminohexanoic acid;
63. 6-(C-β-L-fucopyranoside)-5-aminohexanoic acid;
64. 6-(C-α-L-fucopyranoside)-5-aminohexanoic acid;
65. 1-(C-β-D-fucopyranoside)hexane-2,6-diol;
66. 1-(C-α-D-fucopyranoside)hexane-2,6-diol;
67. 1-(C-β-L-fucopyranoside)hexane-2,6-diol;
68. 1-(C-α-L-fucopyranoside)hexane-2,6-diol;
69. 5-(C-β-D-fucopyranoside)-4-ketopentanoic acid;
70. 5-(C-α-D-fucopyranoside)-4-ketopentanoic acid;
71. 5-(C-β-L-fucopyranoside)hexane-2,6-diol)-4-ketopentanoic acid;
72. 5-(C-α-L-fucopyranoside)hexane-2,6-diol)-4-ketopentanoic acid;
73. 5-(C-β-D-fucopyranoside)-4-hydroxypentanoic acid;
74. 5-(C-α-D-fucopyranoside)-4-hydroxypentanoic acid;
75. 5-(C-β-L-fucopyranoside)-4-hydroxypentanoic acid;
76. 5-(C-α-L-fucopyranoside)-4-hydroxypentanoic acid;
77. 5-(C-β-D-fucopyranoside)-4-aminopentanoic acid;
78. 5-(C-α-D-fucopyranoside)-4-aminopentanoic acid;
79. 5-(C-β-L-fucopyranoside)-4-aminopentanoic acid;
80. 5-(C-α-L-fucopyranoside)-4-aminopentanoic acid;
81. 1-(C-β-D-fucopyranoside)pentane-2,5-diol;
82. 1-(C-α-D-fucopyranoside)pentane-2,5-diol;
83. 1-(C-β-L-fucopyranoside)pentane-2,5-diol;
84. 1-(C-α-L-fucopyranoside)pentane-2,5-diol;
85. 1-(C-β-D-glucopyranosyl)-2-hydroxypropane;
86. 1-(C-α-D-glucopyranosyl)-2-hydroxypropane;
87. 1-(C-β-D-glucopyranosyl)-2-aminopropane;
88. 1-(C-α-D-glucopyranosyl)-2-aminopropane;
89. 1-(C-β-D-glucopyranosyl)-2-(phenylamino)propane;
90. 1-(C-α-D-glucopyranosyl)-2-(phenylamino)propane;
91. ethyl ester of 3-methyl-4-(C-β-D-glucopyranosyl)butyric acid;
92. ethyl ester of 3-methyl-4-(C-α-D-glucopyranosyl)butyric acid;
93. 6-(C-β-D-glucopyranosyl)-5-ketohexanoic acid;
94. 6-(C-α-D-glucopyranosyl)-5-ketohexanoic acid;
95. 6-(C-β-D-glucopyranosyl) -5-hydroxyhexanoic acid;
96. 6-(C-α-D-glucopyranosyl)-5-hydroxyhexanoic acid;
97. 6-(C-β-D-glucopyranosyl)-5-aminohexanoic acid;
98. 6-(C-α-D-glucopyranosyl)-5-aminohexanoic acid;
99. 6-(C-β-D-glucopyranosyl)-5-(phenylamino)hexanoic acid;
100. 6-(C-α-D-glucopyranosyl)-5-(phenylamino)hexanoic acid;
101. 1-(C-β-D-glucopyranosyl)hexane-2,6-diol;
102. 1-(C-α-D-glucopyranosyl)hexane-2,6-diol;
103. 6-(C-β-D-glucopyranosyl)-5-ketopentanoic acid;
104. 6-(C-α-D-glucopyranosyl)-5-ketopentanoic acid;
105. 6-(C-β-D-glucopyranosyl)-5-hydroxypentanoic acid;
106. 6-(C-α-D-glucopyranosyl)-5-hydroxypentanoic acid;
107. 6-(C-β-D-glucopyranosyl)-5-aminopentanoic acid;
108. 6-(C-α-D-glucopyranosyl)-5-hydroxypentanoic acid;
109. 6-(C-β-D-glucopyranosyl)-5-(phenylamino)pentanoic acid;
110. 6-(C-α-D-glucopyranosyl)-5-(phenylamino)pentanoic acid;
111. 1-(C-β-D-glucopyranosyl)pentane-2,5-diol;
112. 1-(C-α-D-glucopyranosyl)pentane-2,5-diol;
113. 1-(C-β-D-galactopyranosyl)-2-hydroxypropane;
114. 1-(C-α-D-galactopyranosyl)-2-hydroxypropane;
115. 1-(C-β-D-galactopyranosyl)-2-aminopropane;
116. 1-(C-α-D-galactopyranosyl)-2-aminopropane;
117. 1-(C-β-D-galactopyranosyl)-2-(phenylamino)propane;
118. 1-(C-α-D-galactopyranosyl)-2-(phenylamino)propane;
119. ethyl ester of 3-methyl-4-(C-β-D-galactopyranosyl)butyric acid;
120. ethyl ester of 3-methyl-4-(C-α-D-galactopyranosyl)butyric acid;
121. 6-(C-β-D-galactopyranosyl)-5-ketohexanoic acid;
122. 6-(C-α-D-galactopyranosyl)-5-ketohexanoic acid;
123. 6-(C-β-D-galactopyranosyl)-5-hydroxyhexanoic acid;
124. 6-(C-α-D-galactopyranosyl)-5-hydroxyhexanoic acid;
125. 6-(C-β-D-galactopyranosyl)-5-aminohexanoic acid;
126. 6-(C-α-D-galactopyranosyl)-5-aminohexanoic acid;
127. 6-(C-β-D-galactopyranosyl)-5-(phenylamino)hexanoic acid;
128. 6-(C-α-D-galactopyranosyl)-5-(phenylamino)hexanoic acid;
129. 1-(C-β**-**D-galactopyranosyl)hexane-2,6-diol;
130. 1-(C-α-D-galactopyranosyl)hexane-2,6-diol;
131. 6-(C-β-D-galactopyranosyl)-5-ketopentanoic acid;
132. 6-(C-α-D-galactopyranosyl)-5-ketopentanoic acid;
133. 6-(C-β-D-galactopyranosyl)-5-hydroxypentanoic acid;
134. 6-(C-α-D-galactopyranosyl)-5-hydroxypentanoic acid;
135. 6-(C-β-D-galactopyranosyl)-5-aminopentanoic acid;
136. 6-(C-α-D-galactopyranosyl)-5-aminopentanoic acid;
137. 6-(C-β**-**D-galactopyranosyl)-5-(phenylamino)-pentanoic acid;
138. 6-(C-α-D-galactopyranosyl)-5-(phenylamino)-pentanoic acid;
139. 1-(C-β-D-galactopyranosyl)pentane-2,6-diol;
140. 1-(C-α-D-galactopyranosyl)pentan-2,6-diol;
141. 1-(C-β-D-fucofuranosyl)propan-2-one;
142. 1-(C-α-D-fucofuranosyl)propan-2-one;
143. 1-(C-β-L-fucofuranosyl)propan-2-one;
144. 1-(C-α-L-fucofuranosyl)propan-2-one;
145. 3'-(acetamido-C-β-D-glucopyranosyl)propan-2'-one;
146. 3'-(acetamido-C-α-D-glucopyranosyl)propan-2'-one;
147. 1-(C-β-D-galactopyranosyl)-2-hydroxypropane;
148. 1-(C-α-D-galactopyranosyl)-2-aminopropane;
149. 1-(acetamido-C-β-D-glucopyranosyl)-2-(phenylamino)propane;
150. 1-(acetamido-C-α-D-glucopyranosyl)-2-(phenylamino)propane;
151. ethyl ester of 3-methyl-4-(acetamido-C-β-D-glucopyranosyl)butyric acid;
152. ethyl ester of 3-methyl-4-(acetamido-C-a-D-glucopyranosyl)butyric acid;
153. 6-(acetamido-C-β-D-glucopyranosyl)-5-ketohexanoic acid;
154. 6-(acetamido-C-α-D-glucopyranosyl)-5-ketohexanoic acid;
155. 6-(acetamido-C-β-D-glucopyranosyl)-5-hydroxyhexanoic acid;
156. 6-(acetamido-C-α-D-glucopyranosyl)-5-hydroxyhexanoic acid;
157. 6-(acetamido-C-β-D-glucopyranosyl)-5-aminohexanoic acid;
158. 6-(acetamido-C-α-D-glucopyranosyl)-5-aminohexanoic acid;
159. 6-(acetamido-C-β-D-glucopyranosyl)-5-(phenylamino)hexanoic acid;
160. 6-(acetamido-C-α-D-glucopyranosyl)-5-(phenylamino)-hexanoic acid;
161. 1-(acetamido-C-β-D-glucopyranosyl)hexane-2,6-diol;
162. 1-(acetamido-C-α-D-glucopyranosyl)hexane-2,6-diol;
163. 6-(acetamido-C-β-D-glucopyranosyl)-5-ketopentanoic acid;
164. 6-(acetamido-C-α-D-glucopyranosyl)-5-ketopentanoic acid;
165. 6-(acetamido-C-β-D-glucopyranosyl)-5-hydroxypentanoic acid;
166. 6-(acetamido-C-α-D-glucopyranosyl)-5-hydroxypentanoic acid;
167. 6-(acetamido-C-β-D-glucopyranosyl)-5-aminopentanoic acid;
168. 6-(acetamido-C-α-D-glucopyranosyl)-5-aminopentanoic acid;
169. 6-(acetamido-C-β-D-glucopyranosyl)-5-(phenylamino)pentanoic acid;
170. 6-(acetamido-C-α-D-glucopyranosyl)-5-(phenylamino)pentanoic acid;
171. 1-(acetamido-C-β-D-glucopyranosyl)pentane-2,5-diol;
172. 1-(acetamido-C-α-D-glucopyranosyl)pentane-2,5-diol.

16. Use according to any one of the preceding claims, **characterized in that** the said derivative is used in an amount representing from 0.0001% to 25% of the total weight of the composition.

17. Use according to Claim 16, **characterized in that** the said derivative is used in an amount representing from 0.001% to 10% of the total weight of the composition.

18. Use according to any one of Claims 8 to 17, **characterized in that** the said C-glycoside derivative is used in combination with at least one depigmenting agent and/or at least one organic photoprotective agent and/or at least one inorganic photoprotective agent.

19. Non-therapeutic cosmetic process for whitening the skin and/or the scalp and/or mucous membranes, comprising the ingestion or the application to the skin and/or scalp and/or mucous membranes of at least one C-glycoside compound corresponding to the formula (I) as defined in any one of the preceding claims.

## Patentansprüche

1. Nichttherapeutische Verwendung mindestens eines C-Gycosidderivats der folgenden Formel (I): 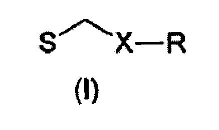 in der Formel:
R bedeutet eine lineare Gruppe mit 1 bis 4 Kohlenstoffatomen, die gegebenenfalls mit -OH, -COOH oder -COOR"₂ substituiert ist, wobei R"₂ eine gesättigte C₁₋₄-Alkylgruppe bedeutet,
X bedeutet eine Gruppe, die unter den folgenden Gruppen ausgewählt ist: 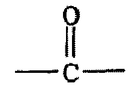 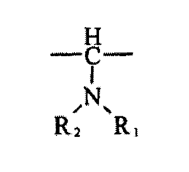 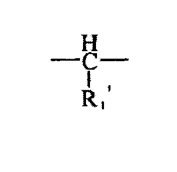 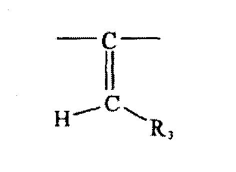 wobei R₁, R₂ und R₃ unabhängig voneinander ein Wasserstoffatom oder eine Gruppe R bedeuten, wobei R die oben angegebenen Bedeutungen aufweist, und R'₁ ein Wasserstoffatom, eine Gruppe -OH oder eine Gruppe R bedeutet, wobei R die oben angegebenen Bedeutungen aufweist, wobei R₁ auch eine C₆₋₁₀-Arylgruppe bedeuten kann,
S bedeutet ein Monosaccharid oder ein Polysaccharid mit bis zu 20 Zuckereinheiten und insbesondere bis zu 6 Zuckereinheiten in Pyranoseform und/oder Furanoseform aus der L- und/oder D-Reihe, wobei das Mono- oder Polysaccharid mit einer Hydroxygruppe, die zwingend frei vorliegt, und gegebenenfalls einer oder mehreren Aminofunktion(en), die gegebenenfalls geschützt sind, substituiert sein kann, und
die Bindung S-CH₂-X bedeutet eine Bindung vom C-anomeren Typ, die α oder β sein kann,
sowie ihrer kosmetisch akzeptablen Salze, ihrer Solvate, wie ihrer Hydrate,
als Mittel zum Bleichen der Haut und/oder als anti-Bräunungsmittel.

2. Verwendung nach Anspruch 1, **dadurch gekennzeichnet, dass**:
• R bedeutet eine lineare C₁₋₄-Gruppe, die gegebenenfalls mit -OH, -COOH oder -COOR"₂ substituiert ist, wobei R"₂ eine gesättigte C₁₋₄-Alkylgruppe bedeutet,
• S bedeutet ein Monosaccharid, wie es in Anspruch 1 beschrieben ist, und
• X bedeutet eine Gruppe, die unter -CO-, -CH(OH)-, - CH(NH₂)-, -CH(NHCH₂CH₂CH₂OH)-, -CH(NHPh)- und
• -CH(CH₃)- ausgewählt ist.

3. Verwendung nach Anspruch 2, **dadurch gekennzeichnet, dass** R eine lineare C₁₋₃-Gruppe bedeutet, die gegebenenfalls mit -OH, -COOH oder -COOR"₂ substituiert ist, wobei R"₂ eine gesättigte C₁₋₄-Alkylgruppe ist.

4. Verwendung nach Anspruch 2 oder 3, **dadurch gekennzeichnet, dass** X eine Gruppe bedeutet, die unter -CO-, -CH(OH)-, -CH(NH₂)- ausgewählt ist.

5. Verwendung nach einem der vorhergehenden Ansprüche, **dadurch gekennzeichnet, dass** S ein Monosaccharid bedeutet, dass unter D-Glucose, D-Xylose, N-Acetyl-D-glucosamin oder L-Fucose und insbesondere D-Xylose ausgewählt ist.

6. Verwendung nach einem der vorhergehenden Ansprüche, **dadurch gekennzeichnet, dass** R die Methylgruppe bedeutet.

7. Verwendung nach einem der vorhergehenden Ansprüche, um die Farbe gebräunter Haut aufzuhellen und/oder zu bleichen und/oder gleichförmig zu machen, der Bildung von bräunlichen Pigmentflecken und/oder Altersflecken und/oder Sommersprossen vorzubeugen und/oder diese zu beseitigen.

8. Verwendung mindestens eines C-Gycosidderivats der folgenden Formel (I): 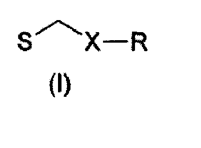 wobei in der Formel:
• R bedeutet lineare eine C₁₋₄-Alkylgruppe, die gegebenenfalls mit -OH, -COOH oder -COOR"₂ substituiert ist, wobei R"₂ eine gesättigte C₁₋₄-Alkylgruppe bedeutet,
• X bedeutet eine Gruppe, die unter den folgenden Gruppen ausgewählt ist: 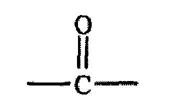 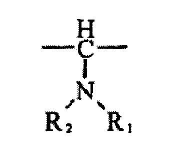 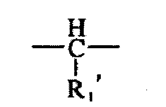 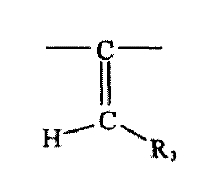 wobei R₁, R₂ und R₃ unabhängig voneinander ein Wasserstoffatom oder eine Gruppe R bedeuten, wobei R die oben angegebenen Bedeutungen aufweist, und R'₁ ein Wasserstoffatom, eine Gruppe -OH oder eine Gruppe R bedeutet, wobei R die oben angegebenen Bedeutungen aufweist, wobei R₁ auch eine C₆₋₁₀-Arylgruppe bedeuten kann,
• S bedeutet ein Monosaccharid oder ein Polysaccharid mit bis zu 20 Zuckereinheiten und insbesondere bis zu 6 Zuckereinheiten in Pyranoseform und/oder Furanoseform aus der L- und/oder D-Reihe, wobei das Mono- oder Polysaccharid mit einer Hydroxygruppe, die zwingend frei vorliegt, und gegebenenfalls einer oder mehreren Aminofunktion(en), die gegebenenfalls geschützt sind, substituiert sein kann, und
• die Bindung S-CH₂-X bedeutet eine Bindung vom C-anomeren Typ, die α oder β sein kann,
sowie ihrer kosmetisch akzeptablen Salze, ihrer Solvate, wie ihrer Hydrate,
für die Herstellung einer Zusammensetzung, die für die Behandlung von Pigmentierungsstörungen vorgesehen ist.

9. Verwendung nach Anspruch 8, **dadurch gekennzeichnet, dass**:
• R bedeutet eine lineare C₁₋₄-Gruppe, die gegebenenfalls mit -OH, -COOH oder -COOR"₂ substituiert ist, wobei R"₂ eine gesättigte C₁₋₄-Alkylgruppe bedeutet,
• S bedeutet ein Monosaccharid, wie es in Anspruch 8 beschrieben ist,
• X bedeutet eine Gruppe, die unter -CO-, -CH(OH)-,
• -CH(NH₂)-, -CH(NHCH₂CH₂CH₂OH)-, -CH(NHPh)- und
• -CH(CH₃)- ausgewählt ist.

10. Verwendung nach Anspruch 9, **dadurch gekennzeichnet, dass** R eine lineare C₁₋₃-Gruppe bedeutet, die gegebenenfalls mit -OH, -COOH oder -COOR"₂ substituiert ist, wobei R"₂ eine gesättigte C₁₋₄-Alkylgruppe ist.

11. Verwendung nach Anspruch 9 oder 10, **dadurch gekennzeichnet, dass** X eine Gruppe bedeutet, die unter -CO-, -CH(OH)-, -CH(NH₂)- ausgewählt ist.

12. Verwendung nach einem der Ansprüche 8 bis 11, **dadurch gekennzeichnet, dass** S ein Monosaccharid bedeutet, dass unter D-Glucose, D-Xylose, N-Acetyl-D-glucosamin oder L-Fucose und insbesondere D-Xylose ausgewählt ist.

13. Verwendung nach einem der Ansprüche 8 bis 12, **dadurch gekennzeichnet, dass** R die Methylgruppe bedeutet.

14. Verwendung nach einem der Ansprüche 8 bis 13, **dadurch gekennzeichnet, dass** die Zusammensetzung für die Behandlung von Melasmen der Unterarme, idiopathischen Melasmen, mit der Schwangerschaft oder einer östro-progestativen Verhütung zusammenhängenden Hyperpigmentierungen, Puva-Lentigines, zufälligen Hyperpigmentierungen, durch Leukodermien verursachten Hyperpigmentierungen und Vitiligo vorgesehen ist.

15. Verwendung nach einem der vorhergehenden Ansprüche, **dadurch gekennzeichnet, dass** das Derivat ausgewählt ist unter:
1. C-β-D-Xylopyranosid-n-propan-2-on;
2. C-α-D-Xylopyranosid-n-propan-2-on;
3. 1-[2-(3-Hydroxy-propylamino)-propyl]-C-β-D-xylopyranose;
4. 1-[2-(3-Hydroxy-propylamino)-propyl]-C-α-D-xylopyranose;
5. C-β-D-Xylopyranosid-2-hydroxy-propan;
6. C-α-D-Xylopyranosid-2-hydroxy-propan;
7. C-β-D-Xylopyranosid-2-amino-propan;
8. C-α-D-Xylopyranosid-2-amino-propan;
9. C-β-D-Xylopyranosid-2-phenylamino-propan;
10. C-α-D-Xylopyranosid-2-phenylamino-propan;
11. Ethylester der 3-Methyl-4-(C-β-D-xylopyranosid)-buttersäure;
12. Ethylester der 3-Methyl-4-(C-α-D-xylopyranosid)-buttersäure;
13. 6-(C-β-D-Xylopyranosid)-5-keto-hexansäure;
14. 6-(C-α-D-Xylopyranosid)-5-keto-hexansäure;
15. 6-(C-β-D-Xylopyranosid)-5-hydroxy-hexansäure;
16. 6-(C-α-D-Xylopyranosid)-5-hydroxy-hexansäure;
17. 6-(C-β-D-Xylopyranosid)-5-amino-hexansäure;
18. 6-(C-α-D-Xylopyranosid)-5-amino-hexansäure;
19. 6-(C-β-D-Xylopyranosid)-5-phenylamino-hexansäure;
20. 6-(C-α-D-Xylopyranosid)-5-phenylamino-hexansäure;
21. 1-(C-β-D-Xylopyranosid)-hexan-2,6-diol;
22. 1-(C-α-D-Xylopyranosid)-hexan-2,6-diol;
23. 5-(C-β-D-Xylopyranosid)-4-keto-pentansäure;
24. 5-(C-α-D-Xylopyranosid)-4-keto-pentansäure;
25. 5-(C-β-D-Xylopyranosid)-4-hydroxy-pentansäure;
26. 5-(C-α-D-Xylopyranosid)-4-hydroxy-pentansäure;
27. 5-(C-β-D-Xylopyranosid)-4-amino-pentansäure;
28. 5-(C-α-D-Xylopyranosid)-4-amino-pentansäure;
29. 5-(C-β-D-Xylopyranosid)-4-phenylamino-pentansäure;
30. 5-(C-α-D-Xylopyranosid)-4-phenylamino-pentansäure;
31. 1-(C-β-D-Xylopyranosid)-pentan-2,5-diol;
32. 1-(C-α-D-Xylopyranosid)-pentan-2,5-diol;
33. 1-(C-β-D-Fucopyranosid)-propan-2-on;
34. 1-(C-α-D-Fucopyranosid)-propan-2-on;
35. 1-(C-β-L-Fucopyranosid)-propan-2-on;
36. 1-(C-α-L-Fucopyranosid)-propan-2-on;
37. 1-(C-β-D-Fucopyranosid)-2-hydroxy-propan;
38. 1-(C-α-D-Fucopyranosid)-2-hydroxy-propan;
39. 1-(C-β-L-Fucopyranosid)-2-hydroxy-propan;
40. 1-(C-α-L-Fucopyranosid)-2-hydroxy-propan;
41. 1-(C-β-D-Fucopyranosid)-2-amino-propan;
42. 1-(C-α-D-Fucopyranosid)-2-amino-propan;
43. 1-(C-β-L-Fucopyranosid)-2-amino-propan;
44. 1-(C-α-L-Fucopyranosid)-2-amino-propan;
45. 1-(C-β-D-Fucopyranosid)-2-phenylamino-propan;
46. 1-(C-α-D-Fucopyranosid)-2-phenylamino-propan;
47. 1-(C-β-L-Fucopyranosid)-2-phenylamino-propan;
48. 1-(C-α-L-Fucopyranosid)-2-phenylamino-propan;
49. Ethylester von 3-Methyl-4-(C-β-D-fucopyranosid)-buttersäure;
50. Ethylester von 3-Methyl-4-(C-α-D-fucopyranosid)-buttersäure;
51. Ethylester von 3-Methyl-4-(C-β-L-fucopyranosid)-buttersäure;
52. Ethylester von 3-Methyl-4-(C-α-L-fucopyranosid)-buttersäure;
53. 6-(C-β-D-Fucopyranosid)-5-keto-hexansäure;
54. 6-(C-α-D-Fucopyranosid)-5-keto-hexansäure;
55. 6-(C-β-L-Fucopyranosid)-5-keto-hexansäure;
56. 6-(C-α-L-Fucopyranosid)-5-keto-hexansäure;
57. 6-(C-β-D-Fucopyranosid)-5-hydroxy-hexansäure;
58. 6-(C-α-D-Fucopyranosid)-5-hydroxy-hexansäure;
59. 6-(C-β-L-Fucopyranosid)-5-hydroxy-hexansäure;
60. 6-(C-α-L-Fucopyranosid)-5-hydroxy-hexansäure;
61. 6-(C-β-D-Fucopyranosid)-5-amino-hexansäure;
62. 6-(C-α-D-Fucopyranosid)-5-amino-hexansäure;
63. 6-(C-β-L-Fucopyranosid)-5-amino-hexansäure;
64. 6-(C-α-L-Fucopyranosid)-5-amino-hexansäure;
65. 1-(C-β-D-Fucopyranosid)-hexan-2,6-diol;
66. 1-(C-α-D-Fucopyranosid)-hexan-2,6-diol;
67. 1-(C-β-L-Fucopyranosid)-hexan-2,6-diol;
68. 1-(C-α-L-Fucopyranosid)-hexan-2,6-diol;
69. 5-(C-β-D-Fucopyranosid)-4-keto-pentansäure;
70. 5-(C-α-D-Fucopyranosid)-4-keto-pentansäure;
71. 5-(C-β-L-Fucopyranosid)-hexan-2,6-diol)-4-ketopentansäure;
72. 5-(C-α-L-Fucopyranosid)-hexan-2,6-diol)-4-ketopentansäure;
73. 5-(C-β-D-Fucopyranosid)-4-hydroxy-pentansäure;
74. 5-(C-α-D-Fucopyranosid)-4-hydroxy-pentansäure;
75. 5-(C-β-L-Fucopyranosid)-4-hydroxy-pentansäure;
76. 5-(C-α-L-Fucopyranosid)-4-hydroxy-pentansäure;
77. 5-(C-β-D-Fucopyranosid)-4-amino-pentansäure;
78. 5-(C-α-D-Fucopyranosid)-4-amino-pentansäure
79. 5-(C-β-L-Fucopyranosid)-4-amino-pentansäure;
80. 5-(C-α-L-Fucopyranosid)-4-amino-pentansäure;
81. 1-(C-β-D-Fucopyranosid)-pentan-2,5-diol;
82. 1-(C-α-D-Fucopyranosid)-pentan-2,5-diol;
83. 1-(C-β-L-Fucopyranosid)-pentan-2,5-diol;
84. 1-(C-α-L-Fucopyranosid)-pentan-2,5-diol;
85. 1-(C-β-D-Glucopyranosyl)-2-hydroxyl-propan;
86. 1-(C-α-D-Glucopyranosyl)-2-hydroxyl-propan;
87. 1-(C-β-D-Glucopyranosyl)-2-amino-propan;
88. 1-(C-α-D-Glucopyranosyl)-2-amino-propan;
89. 1-(C-β-D-Glucopyranosyl)-2-phenylamino-propan;
90. 1-(C-α-D-Glucopyranosyl)-2-phenylamino-propan;
91. Ethylester von 3-Methyl-4-(C-β-D-Glucopyranosyl)-buttersäure;
92. Ethylester von 3-Methyl-4-(C-α-D-Glucopyranosyl)-buttersäure;
93. 6-(C-β-D-Glucopyranosyl)-5-keto-hexansäure;
94. 6-(C-α-D-Glucopyranosyl)-5-keto-hexansäure;
95. 6-(C-β-D-Glucopyranosyl)-5-hydroxy-hexansäure;
96. 6-(C-α-D-Glucopyranosyl)-5-hydroxy-hexansäure;
97. 6-(C-β-D-Glucopyranosyl)-5-amino-hexansäure;
98. 6-(C-α-D-Glucopyranosyl)-5-amino-hexansäure;
99. 6-(C-β-D-Glucopyranosyl)-5-phenylamino-hexansäure;
100. 6-(C-α-D-Glucopyranosyl)-5-phenylamino-hexansäure;
101. 1-(C-β-D-Glucopyranosyl)-hexan-2,6-diol;
102. 1-(C-α-D-Glucopyranosyl)-hexan-2,6-diol;
103. 6-(C-β-D-Glucopyranosyl)-5-keto-pentansäure;
104. 6-(C-α-D-Glucopyranosyl)-5-keto-pentansäure;
105. 6-(C-β-D-Glucopyranosyl)-5-hydroxy-pentansäure;
106. 6-(C-α-D-Glucopyranosyl)-5-hydroxy-pentansäure;
107. 6-(C-β-D-Glucopyranosyl)-5-amino-pentansäure;
108. 6-(C-α-D-Glucopyranosyl)-5-hydroxy-pentansäure;
109. 6-(C-β-D-Glucopyranosyl)-5-phenylamino-pentansäure;
110. 6-(C-α-D-Glucopyranosyl)-5-phenylamino-pentansäure;
111. 1-(C-β-D-Glucopyranosyl)-pentan-2,5-diol;
112. 1-(C-α-D-Glucopyranosyl)-pentan-2,5-diol;
113. 1-(C-β-D-Galactopyranosyl)-2-hydroxy-propan;
114. 1-(C-α-D-Galactopyranosyl)-2-hydroxy-propan;
115. 1-(C-β-D-Galactopyranosyl)-2-amino-propan;
116. 1-(C-α-D-Galactopyranosyl)-2-amino-propan;
117. 1-(C-β-D-Galactopyranosyl)-2-phenylamino-propan;
118. 1-(C-α-D-Galactopyranosyl)-2-phenylamino-propan;
119. Ethylester von 3-Methyl-4-(C-β-D-galactopyranosyl)-buttersäure;
120. Ethylester von 3-Methyl-4-(C-α-D-galactopyranosyl)-buttersäure;
121. 6-(C-β-D-Galactopyranosyl)-5-keto-hexansäure;
122. 6-(C-α-D-Galactopyranosyl)-5-keto-hexansäure;
123. 6-(C-β-D-Galactopyranosyl)-5-hydroxy-hexansäure;
124. 6-(C-α-D-Galactopyranosyl)-5-hydroxy-hexansäure;
125. 6-(C-β-D-Galactopyranosyl)-5-amino-hexansäure;
126. 6-(C-α-D-Galactopyranosyl)-5-amino-hexansäure;
127. 6-(C-β-D-Galactopyranosyl)-5-phenylamino-hexansäure;
128. 6-(C-α-D-Galactopyranosyl)-5-phenylamino-hexansäure;
129. 1-(C-β-D-Galactopyranosyl)-hexan-2,6-diol;
130. 1-(C-α-D-Galactopyranosyl)-hexan-2,6-diol;
131. 6-(C-β-D-Galactopyranosyl)-5-keto-pentansäure;
132. 6-(C-α-D-Galactopyranosyl)-5-keto-pentansäure;
133. 6-(C-β-D-Galactopyranosyl)-5-hydroxy-pentansäure;
134. 6-(C-α-D-Galactopyranosyl)-5-hydroxy-pentansäure;
135. 6-(C-β-D-Galactopyranosyl)-5-amino-pentansäure;
136. 6-(C-α-D-Galactopyranosyl)-5-amino-pentansäure;
137. 6-(C-β-D-Galactopyranosyl)-5-phenylamino-pentansäure;
138. 6-(C-α-D-Galactopyranosyl)-5-phenylamino-pentansäure;
129. 1-(C-β-D-Galactopyranosyl)-pentan-2,6-diol;
140. 1-(C-α-D-Galactopyranosyl)-pentan-2,6-diol;
141. 1-(C-β-D-Fucofuranosyl)-propan-2-on;
142. 1-(C-α-D-Fucofuranosyl)-propan-2-on;
143. 1-(C-β-L-Fucofuranosyl)-propan-2-on;
144. 1-(C-α-L-Fucofuranosyl)-propan-2-on;
145. 3'-(Acetamido-C-β-D-glucopyranosyl)-propan-2'-on;
146. 3'-(Acetamido-C-α-D-glucopyranosyl)-propan-2'-on;
147. 1-(C-β-D-Galactopyranosyl)-2-hydroxy-propan;
148. 1-(C-α-D-Galactopyranosyl)-2-amino-propan;
149. 1-(Acetamido-C-β-D-glucopyranosyl)-2-phenylaminopropan;
150. 1-(Acetamido-C-α-D-glucopyranosyl)-2-phenylaminopropan;
151. Ethylester von 3-Methyl-4-(acetamido-C-β-D-glucopyranosyl)-buttersäure;
152. Ethylester von 3-Methyl-4-(acetamido-C-α-D-glucopyranosyl)-buttersäure;
153. 6-(Acetamido-C-β-D-glucopyranosyl)-5-keto-hexansäure;
154. 6-(Acetamido-C-α-D-glucopyranosyl)-5-keto-hexansäure;
155. 6-(Acetamido-C-β-D-glucopyranosyl)-5-hydroxyhexansäure;
156. 6-(Acetamido-C-α-D-glucopyranosyl)-5-hydroxyhexansäure;
157. 6-(Acetamido-C-β-D-glucopyranosyl)-5-amino-hexansäure;
158. 6-(Acetamido-C-α-D-glucopyranosyl)-5-amino-hexansäure;
159. 6-(Acetamido-C-β-D-glucopyranosyl)-5-phenylaminohexansäure;
160. 6-(Acetamido-C-α-D-glucopyranosyl)-5-phenylaminohexansäure;
161. 1-(Acetamido-C-β-D-glucopyranosyl)-hexan-2,6-diol;
162. 1-(Acetamido-C-α-D-glucopyranosyl)-hexan-2,6-diol;
163. 6-(Acetamido-C-β-D-glucopyranosyl)-5-keto-pentansäure;
164. 6-(Acetamido-C-α-D-glucopyranosyl)-5-keto-pentansäure;
165. 6-(Acetamido-C-β-D-glucopyranosyl)-5-hydroxypentansäure;
166. 6-(Acetamido-C-α-D-glucopyranosyl)-5-hydroxypentansäure;
167. 6-(Acetamido-C-β-D-glucopyranosyl)-5-amino-pentansäure;
168. 6-(Acetamido-C-α-D-glucopyranosyl)-5-amino-pentansäure;
169. 6-(Acetamido-C-β-D-glucopyranosyl)-5-phenylaminopentansäure;
170. 6-(Acetamido-C-α-D-glucopyranosyl)-5-phenylaminopentansäure;
171. 1-(Acetamido-C-β-D-glucopyranosyl)-pentan-2, 5-diol;
172. 1-(Acetamido-C-α-D-glucopyranosyl)-pentan-2,5-diol.

16. Verwendung nach einem der Ansprüche 8 bis 15, **dadurch gekennzeichnet, dass** das Derivat in einer Menge von 0,0001 bis 25 % des Gesamtgewichts der Zusammensetzung verwendet wird.

17. Verwendung nach Anspruch 16, **dadurch gekennzeichnet, dass** das Derivat in einer Menge von 0,001 bis 10 % des Gesamtgewichts der Zusammensetzung verwendet wird.

18. Verwendung nach einem der Ansprüche 8 bis 17, **dadurch gekennzeichnet, dass** das C-Gycosidderivat mit mindestens einem Depigmentierungsmittel und/oder mindestens einem organischen Lichtschutzmittel und/oder mindestens einem anorganischen Lichtschutzmittel kombiniert wird.

19. Nichttherapeutisches kosmetisches Verfahren zur Aufhellung der Haut und/oder der Kopfhaut und/oder der Schleimhäute, das das Einnehmen oder Auftragen auf die Haut und/oder die Kopfhaut und/oder die Schleimhäute mindestens eines C-Glycosidderivats der Formel (I) umfasst, wie es in einem der vorhergehenden Ansprüche definiert ist.
